# EUROPEAN PATENT APPLICATION

(11) **EP 0 123 950 A2**
(43) Date of publication of application: **07.11.1984**
(21) Application number: 84103670.0
(22) Date of filing: 03.04.1984
(51) Int. Cl.: C07C 103/52, A61K 37/02

(54) **Intermediates for thymosin alpha 1 and desacetylthymosin alpha 1**

(30) Priority: 04.04.1983 US 482114
(71) Applicant: F. HOFFMANN-LA ROCHE & CO. Aktiengesellschaft, 4002 Basel (CH)
(72) Inventor: Felix, Arthur Martin, West Caldwell, N.J. (US); Meienhofer, Johannes Arnold, Upper Montclair, N.J. (US); Trzeciak, Arnold, Dr., Schopfheim (DE); Gillessen, Dieter, Dr., Pratteln (CH); Studer, Rolf, Dr., Bottmingen (CH)
(74) Representative: Lederer, Franz, Dr.

(57) **Abstract**

An improved solution phase synthesis of thymosin a, and desacetyl thymosin a, by couplin t-Boc side chain protected amino terminal decapeptide and carboxy terminal octadecapeptide and intermediates therefor are disclosed.

## Description

The isolation of biologically important peptides from the thymus gland has been studied extensively in the last few years. Several peptides have been shown to play certain roles in T-cell maturation. Thymosin α1, a highly acidic octacosapeptide which is acetylated at the amino terminus isolated from calf thymus gland has been reported to exhibit biological activities related to the development of thymus- dependent lymphocytes (T-cells). Desacetyl thymosin a₁ has been reported to exhibit the same biological activity as thymosin a₁ and further can act as an intermediate in the preparation of thymosin a₁.

The isolation and characterization of thymosin a₁ is disclosed in U.S.P. 4,079,127. Synthesis of this peptide by solution and solid phase procedures is disclosed in U.S.P. 4,148,788. An alternate solution phase synthesis for thymosin a₁ employing a different synthetic strategy was described by Birr and Stollenwerk, Angew. Chem. Int. Ed. Engl. 18, 394 (1979).

A solid phase synthesis of desacetyl thymosin a. was described by Merrifield et al. at the Fifteenth European Peptide Symposium (Poland), September 1978 and was published in the Proceedings of the Fifteenth European Peptide Symposium, June 1979. It was also described by Merrifield in the Alan E. Pierce Award Lecture on Solid Phase Synthesis given June 20, 1979 at the 6th American Peptide Symposium, published December 1979. See also Wong and Merrifield in Biochem. 19, 3233-3238 (1980).

In addition, the biosynthesis of desacetyl thymosin α₁ by a recombinant microorganism constructed by use of recombinant DNA technology has been described in European patent application No. 35454.

The present invention relates to novel intermediates for thymosin α₁ and desacetylthymosin α₁ and to an improved process for the preparation of thymosin α₁ and desacetylthymosin a_{l'} This process involves the use of solution phase peptide synthetic techniques to accomplish the synthesis of several protected fragment precursors which in turn are used to prepare as key intermediates the amino terminus decapeptide and the carboxy terminus octadecapeptide.

More specifically, the present invention relates to compounds of the formula 
R^2-Ser(tBu)-Asp(OtBu)-Ala-Ala-Val-Asp(OtBu)-
Thr(Bu)-Ser(tBu)-Ser(tBu)-Glu(OtBu)-OR^1
wherein _{R}¹ is hydrogen or phenyl,
_{R}² is acetyl or tert. butoxycarbonyl und tBu is tert. butyl,
as well as to compounds of the formula 
R3-Ile-Thr(tBu)-Thr(tBu)-Lys(Boc)-Asp(OtBu)-Leu-
Lys(Boc)-Glu(OtBu)-Lys(Boc)-Lys(Boc)-Glu(Ot-Bu)-
Val-Val-Glu(OtBu)-Glu(OtBu)-Ala-Glu(OtBu)-Asn-OtBu
 wherein R³ is hydrogen or benzyloxycarbonyl,
Boc is tert. butyloxycarbonyl and
tBu is tert. butyl.

The above formulae cover the following compounds: 
Z-Ile-Thr(tBu)-Thr(tBu)-Lys(Boc)-Asp(OtBu)-Leu-
Lys(Boc)-Glu(OtBu)-Lys(Boc)-Lys(Boc)-Glu(OtBu)-Val-
Val-Glu(OtBu)-Glu(OtBu)-Ala-Glu(OtBu)-Asn-OtBu,
 
H-Ile-Thr(tBu)-Thr(tBu)-Lys(Boc)-Asp(OtBu)-Leu-
Lys(Boc)-Glu(OtBu)-Lys(Boc)-Lys(Boc)-Glu(OtBu)-
Val-Val-Glu(OtBu)-Glu(OtBu)-Ala-Glu(OtBu)-Asn-OtBu,

Ac-Ser(tBu)-Asp(OtBu)-Ala-Ala-Val-Asp(OtBu)-Thr(tBu)-
Ser(tBu)-Ser(tBu)-Glu(OtBu)-OC6H5,

Ac-Ser(tBu)-Asp(OtBu)-Ala-Ala-Val-Asp(OtBu)-
Thr(tBu)-Ser(tBu)-Ser(tBu)-Glu(OtBu)-OH
 and 
Boc-Ser(tBu)-Asp(OtBu)-Ala-Val-Val-Asp(OtBu)-
Thr(tBu)-Ser(tBu)-Glu(OtBu)-OH
 wherein Z is benzyloxycarbonyl, tBu is tert. butyl and Ac is acetyl.

A further aspect of the invention involves the use of tert-butyl groups as side chain protecting groups. This allows use of mild acid hydrolysis of the protective groups from the protected precursor compounds to the desired end products and thereby avoids the use of final deprotection with very strong acid such as liquid HF which can cause extensive degradation of the product. The instant process thus provides a highly efficient procedure for producing the desired thymosin a₁ and desacetylthymosin a₁ in higher yields than has heretofore been possible.

### Description of the Drawings

Figures la and 1b set forth the synthesis strategy for thymosin a₁ from seven protected peptide fragments (I-VII).
Figure 2 sets forth the procedure used to synthesize fragment I.
Figure 3 sets forth the procedure used to synthesize fragment II.
Figure 4 sets forth the procedure used to synthesize fragment III.
Figure 5 sets forth the procedure used to synthesize fragment IV.
Figure 6 sets forth the procedure used to synthesize fragment V.
Figure 7 sets forth the procedure used to synthesize fragment VI.
Figure 8 sets forth the procedure used to synthesize fragment VII.

While the synthesis has been described in detail in relation to the preparation of thymosin α₁, the same overall strategy is employed to produce desacetyl thymosin α₁ with the minor modification that Boc-Ser(t-Bu)-Asp-(OtBu)-Ala-OSu is substituted for fragment VII.

### Description of the Invention

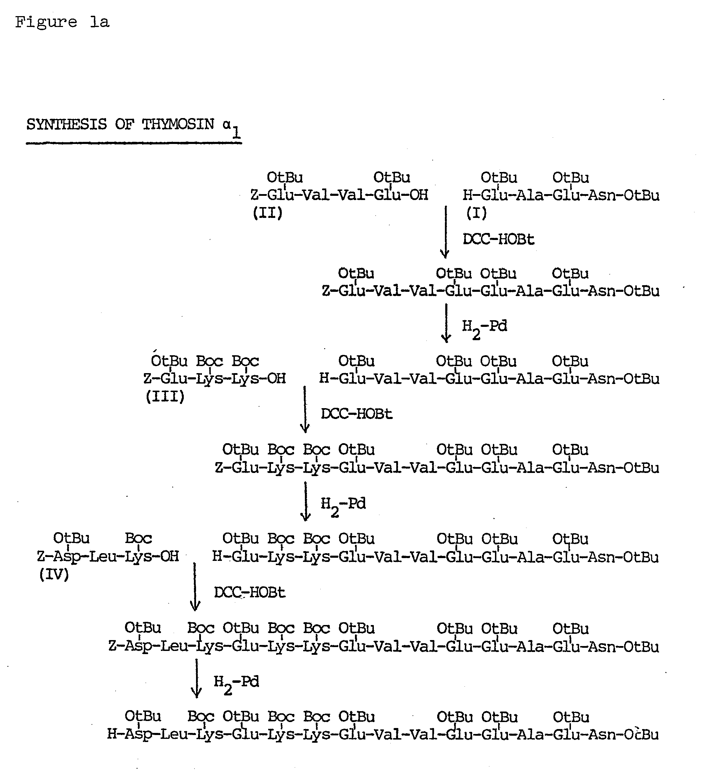

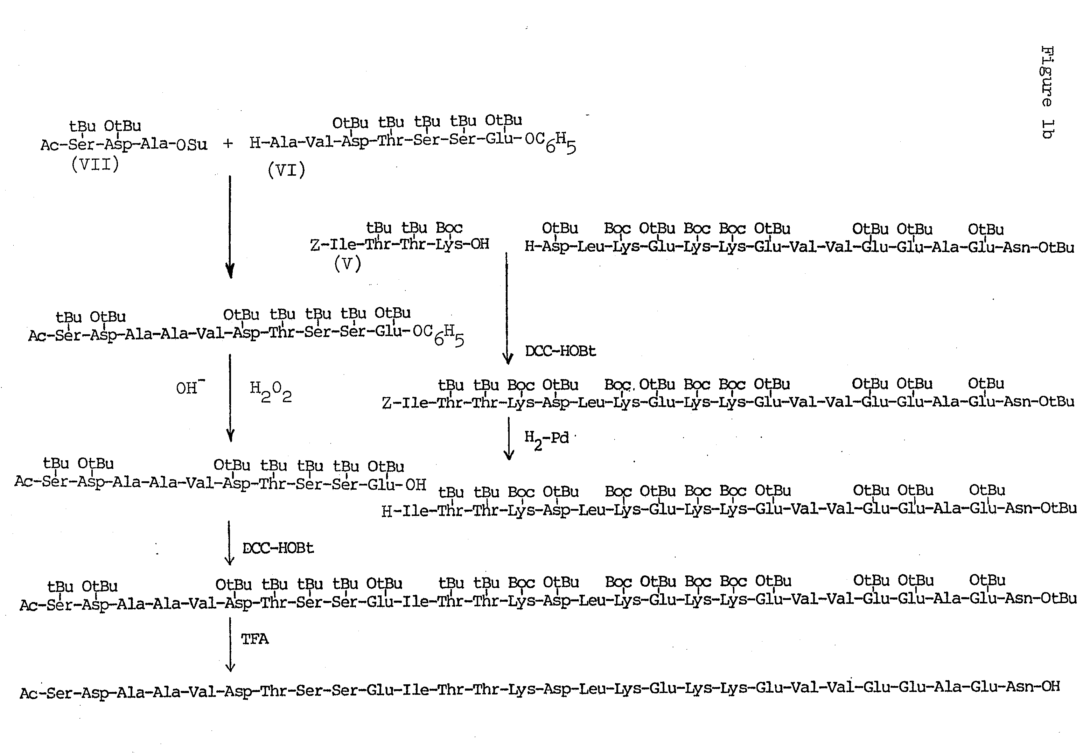

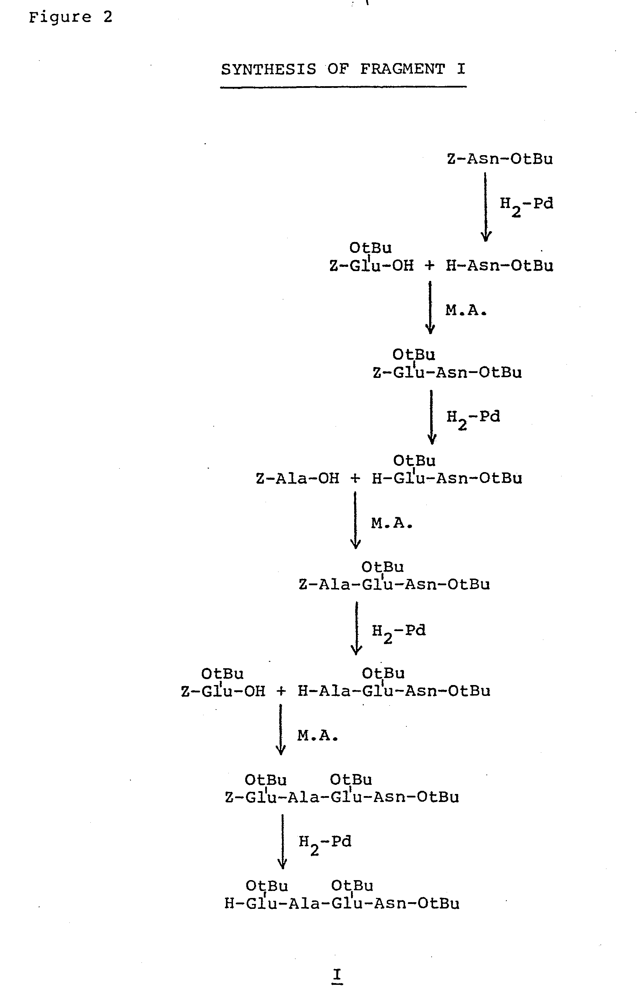

As seen from the synthetic strategy set forth in Figures la and 1b the procedure utilized for the synthesis of thymosin a< and desacetylthymosin α₁ required the synthesis of 7 fragments (I-VII). The synthetic approaches to the 7 fragments are outlined in Figures 2-8 respectively.

Fragment I was prepared by stepwise chain elongation using the mixed anhydride (M.A.) procedure at -15°C with careful temperature control. As seen in the scheme of Figure 2 mixed anhydride coupling (isobutylchloroformate) of Z-Glu(OtBu)-OH with H-Asn-OtBu provided Z-Glu(OtBu)-Asn-OtBu. Catalytic hydrogenation (10% Pd/C) of this compound cleaved the N-terminal protective group and the dipeptide was coupled to Z-Ala-OH using mixed anhydride coupling to give Z-Ala-Glu(OtBu)-Asn-OtBu. The N-terminus was again deprotected by catalytic hydrogenation and the tripeptide was coupled with Z-Glu(OtBu)-Oh to give the protected tetrapeptide Z-Glu(OtBu)-Ala-Glu(OtBu)-Asn-OtBu which is deblocked by catalytic hydrogenation to provide the desired tetrapeptide (I).

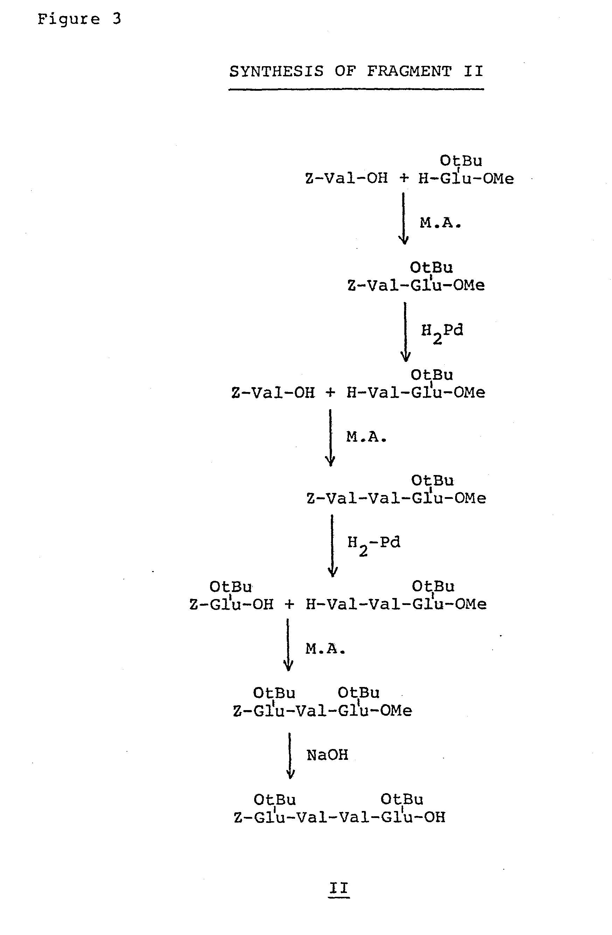

The synthesis of fragment II is depicted in Figure 3. Again all three coupling reactions were carried out by the mixed anhydride method. Thus HC1 H-Glu(OtBu)-OMe was converted to Z-Val-Glu(OtBu)-OMe, which after catalytic hydrogenation for 25 hours to the resulting intermediate H-Val-Glu(OtBu)-OMe was immediately coupled (M.A.) with Z-Val-OH. The resultant protected tripeptide Z-Val-Val-Glu(OtBu)-OMe was then catalytically hydrogenated to produce the corresponding N-terminal free peptide which was coupled (M.A.) with Z-Glu(OtBu)-OH. The resulting protected tetrapeptide was then saponified with 1N NaOH in DMF to provide fragment II.

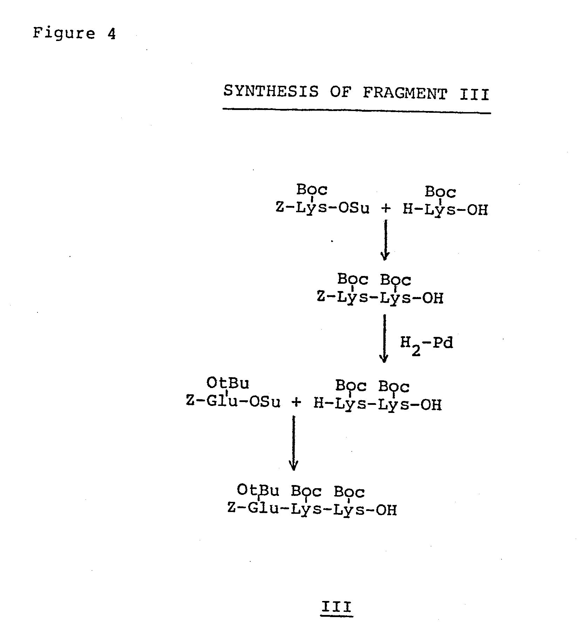

In Figure 4, the synthesis of fragment III is shown. Coupling of Z-Lys(Boc)-OSu with H-Lys(Boc)-OH gave Z-Lys (Boc)-Lys(Boc)-OH as the DCHA salt. Conversion to the free acid using H₂S0₄ in ether was followed by catalytic hydrogenation (5% Pd/BaS0₄) and by coupling with Z-Glu(OtBu)-OSu to yield the desired fragment III Z-Glu(OtBu)-Lys(Boc)₋ Lys(Boc)-OH.

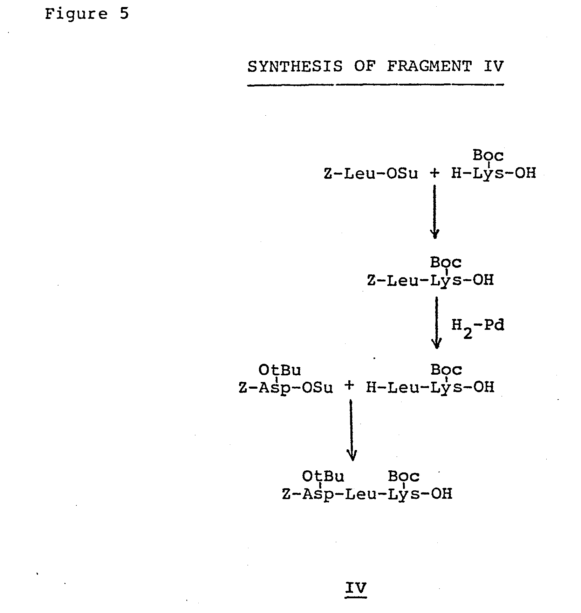

The scheme shown in Figure 5 describes the preparation of fragment IV. Conversion of Z-Leu-OH to Z-Leu-OSu and Z-Asp(OtBu)-OH to Z-Asp(OtBu)-OSu was carried out in a manner known per se using DCC and HOSU. Coupling of Z-Leu-OSu with H-Lys(Boc)-OH provided Z-Leu- Lys(Boc)-OH as the DCHA salt. Conversion to the free amino terminus followed by catalytic hydrogenolysis (Pd/BaS0₄) gave H-Leu-Lys(Boc)-OH. Final coupling of H-Leu-Lys(Boc)-OH with Z-Asp(OtBu)-OSu gave Fragment IV.

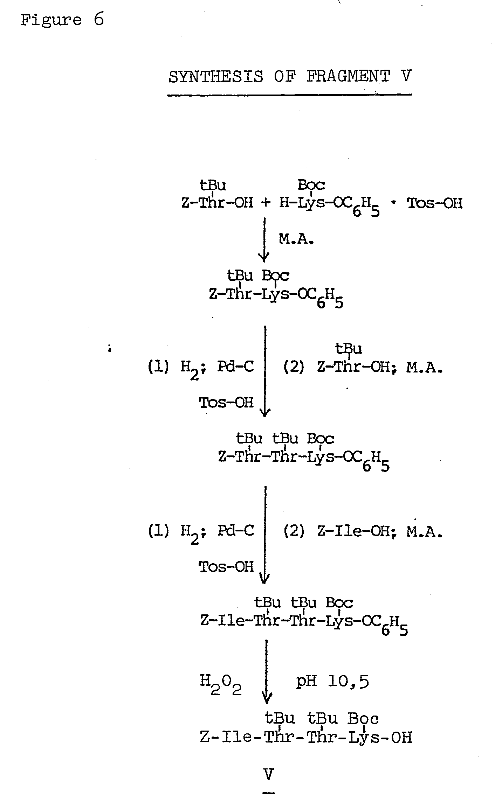

Figure 6 shows the procedure used for the preparation of Fragment V. Z-Lys(Boc)-OH was esterified with phenol and the amino terminus was deblocked by catalytic hydrogenation over 10% Pd/C in the presence of TosOH to form H-Lys(Boc)-OC₆H₅·TosOH. After coupling (M.A.) with Z-Thr(tBu)-OH the resulting dipeptide was catalytically deblocked and coupled with Z-Thr(tBu)-OH to provide the tripeptide Z-Thr(tBu)-ThrEtBu)-Lys(Boc)-OC₆H₅. After another cycle of catalytic deblocking and coupling with Z-Ile-OH (M.A.), the resulting tetrapeptide ester was saponified with dilute, basic hydrogen peroxide to yield the desired fragment V, Z-Ile-Thr(tBu)-Thr(tBu)-Lys(Boc)-OH.

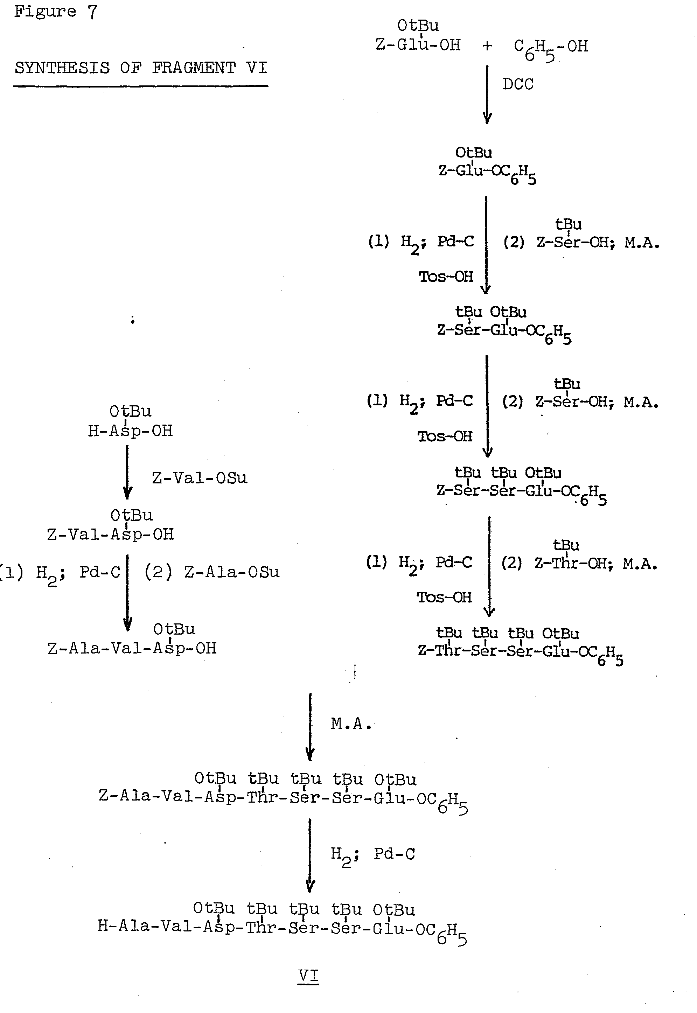

The synthesis of fragment VI is shown in Figure 7. The esterification of Z-Glu(OtBu)-OH with phenol was carried out under conventional conditions and the resulting phenyl ester, after catalytic (Pd/C) deblocking, is coupled (M.A.) with Z-Ser(tBu)-OH to provide the dipeptide Z-Ser(tBu)-GIu(OtBu)-OC₆H₅. Two addition cycles of deblocking and mixed anhydride coupling with Z-Ser(tBu}-OH and Z-Thr(tBu)-OH respectively provided the protected tetrapeptide Z-Thr(tBu)-Ser(tBu)-Ser(tBu)-GIu(GtBu)-aC₆H₅. Catalytic deprotection in the presence of TosOH gave the corresponding free amino terminus tetrapeptide as the tosyl salt, which upon mixed anhydride coupling with Z-Ala-Val-Asp(OtBu)-OH and catalytic deblocking provided the desired heptapeptide VI.

As further seen in Figure 7 the tripeptide coupling partner is built up by reacting Z-Val-OSu with H-Asp(OtBu)-OH, deblocking the resulting dipeptide by catalytic hydrogenation with Pd/C and then coupling with Z-Ala-OSu.

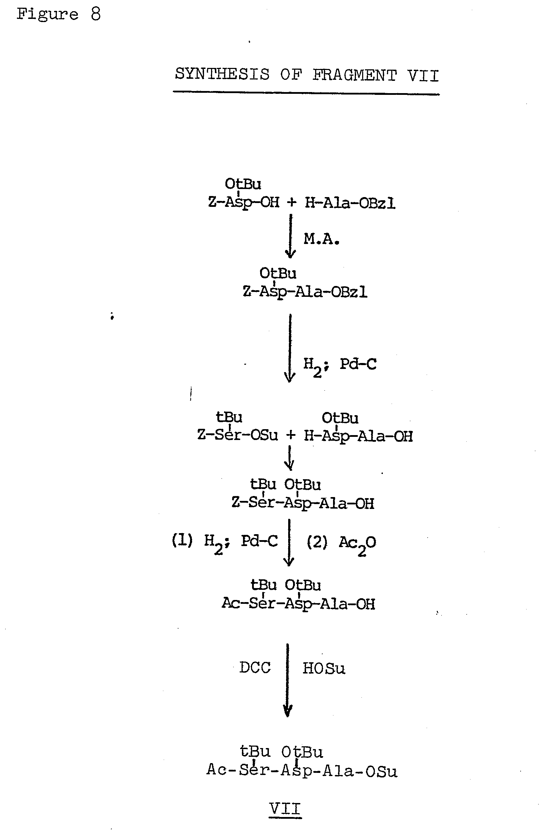

Construction of Fragment VII is set forth in Figure 8. Mixed anhydride coupling of Z-Asp(OtBu)-OH with H-Ala-OBzl produced the protected dipeptide which was deblocked by catalytic hydrogenation with Pd/C in the presence of TosOH. The resulting deblocked dipeptide in the form of the tosyl salt was then coupled to Z-Ser(tBu)-OSu to provide the protected tripeptide Z-Ser(tBu)-Asp(OtBu}-Ala-OH. After beblocking by catalytic hydrogenation the resulting tripeptide can be treated with acetic anhydride to provide the blocked N-terminal acetyl tripeptide. This blocked tripeptide, Ac-Ser(tBu)-Asp(OtBu)-Ala-OH, was then converted to the corresponding succinimide ester to thereby yield the desired Fragment VII.

In the case of the preparation of desacetyl thymosin α₁, the corresponding equivalent Fragment VII consists of Boc-Ser(tBu)-Asp(OtBu)-Ala-OSu.

The following Examples illustrate but do not limit the instant invention. All temperatures are in centigrade. The abbreviations used are well-known to those skilled in the art.

All amino acids are of the L-configuration. TLC was carried out on silica gel G plates and developed with chlorine-TDM. Typically, 2 mg of sample was dissolved in 150 ul of solvent and 3 µl applied to the TLC plate.

### Example 1

### Synthesis of Fragment I (y-t-Butyl-L-glutamyl-L-alanyl-y-t-butyl-L-glutamyl-L-asparagine-t-butyl ester)

### (a) N-Benzyloxycarbonyl-y-t-butyl-L-glutamyl-L-asparagine t-butyl ester

A solution of Z-Glu(OtBu)-OH (212.5 g, 0.63 mol, 1.05 eq.) in DMF (400 ml) was placed in a 2-1 3-necked round bottom flask fitted with a thermometer, mechanical stirrer, dropping funnel and immersed in a dry ice-acetone bath at -15°. The reaction was carried out under an atmosphere of N₂₁ cooled to -15° and precooled (-20°) N-methylmorpholine (95.6 g, 0.945 mol, 1.575 eq.) was added. After stirring for 4 min. at -15°, the reaction mixture was cooled to -25° and precooled (-20°) isobutylchloroformate (86.0 g, 0.63 mol, 1.05 eq.) was added portionwise over a 2 minutes period (temeprature must not exceed -15°). The reaction mixture was stirred for an additional 4 min. at -25° and a precooled (-20°) solution of H-Asn-OtBu (112.9 g, 0.60 mol, 1 eq.) in DMF (440 ml)/CH₂C1₂ (40 ml) was added as rapidly as possible while the temperature was maintained at -15° to -20°. After the addition was complete the reaction mixture was stirred at -15° for 30 min. and at 25° for 2.5 h. The reaction mixture was filtered and the filter cake washed with DMF (2 x 50 ml). The filtrate was evaporated in vacuo and the resultant syrup dissolved in CH₂Cl₂, (2.5 1) and washed with 10% NaHCO₃ (2 x 450 ml), saturated NaCl (1 x 400 ml), 10% citric acid (3 x 450 ml), saturated NaCl (1 x 400 ml). It was dried (MgSO₄), filtered (celite) and evaporated to dryness to give a syrup which solidified on standing: mp 136-140°. Crystallization from isopropanol (1.1 1)/ligroin (3 1) gave 269.9 g (88.6%) of white crystals; mp. 143-145°; 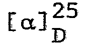 -19.06° (c 1, MeOH); _{Rf} 0.02 (CHCl₃/MeOH/AcOH; 80/2/0.4); ^{R}f₂₅ 0.80 (CHC13/MeOH/AcOH; 80/10/0.4).

### (b) Y-t-Hutyl-L-glutamyl-L-asparagine t-butyl ester

A solution of Z-Glu(OtBu)-Asn-OtBu (40.0 g, 0.079 mol) in MeOH (250 ml) containing 10% Pd/C (5.0 g) was reduced in a Parr hydrogenator until the theoretical amount of H₂ was taken up (and no further uptake of H₂ was observed). The reaction mixture was filtered through celite (topped with a bed of MgSO₄) and evaporated to dryness. A total of 590 g of Z-Glu(OtBu)-Asn-OtBu was reduced and the combined material crystallized by treatment of the residue with MeOH. Evaporation of solvent resulted in the isolation of 397 g (91.4%) of white needles; mp. 117-119°; 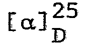 -4.98° (c 1, MeOH); R_{f} 0.25 (CHCl₃/MeOH/AcOH; 85/10/5).

### (c) N-Benzyloxycarbonyl-L-alanyl-y-t-butyl-L-glutamyl-L-asparagine t-butyl ester

A solution of Z-Ala-OH (118.5 g, 0.53 mol, 1.05 eq.) in DMF (400 ml) was placed in a 2-1 3-necked round bottom flask fitted with a thermometer, mechanical stirrer, dropping funnel and immersed in a dry ice-acetone bath at -15°. The reaction was carried out under an atmosphere of N₂, cooled to -15° and precooled (-20°) N-methylmorpholine (80.9 g, 0.80 mol, 1.58 eq.) was added. After stirring for 4 min. at -15°, the reaction mixture was cooled to -25° and precooled (-20°) isobutylchloroformate (72.5 g, 0.53 mol, 1.05 eq.) was added portionwise over a 2 minutes period (temperature must not exceed -15°). The reaction mixture was stirred for an additional 4 min. at -25° and a precooled (-20°) solution of H-Glu(OtBu)-Asn-OtBu (189 g, 0.506 mol) in DMF (350 ml)/CH₂C1₂ (150 ml) was added as rapidly as possible while maintaining -15° to -20° (4 minutes). After the addition was complete the reaction mixture was stirred at -15° for 30 min. and at 25° for 3 h. The reaction mixture was filtered and the filter cake washed with DMF (2 x 50 ml). The filtrate was evaporated in vacuo and the resultant syrup immediately poured into H₂0 (3.5 1). The suspension was stirred for 18 h and the product collected by filtration and washed with H₂O (2 x 500 ml) and dried in vacuo. A total of 575.6 g (98.3%) was prepared in 2 batches. Recrystallization from EtOH (3.5 1) _{H20} (5 1) gave 533.4 g (91.1%); mp. 174-176°; 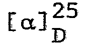 -35.48° (c 1, MeOH); R_{f} 0.25 (CHC1_{3/}MeOH/AcOH; 80/20/5).

### (d) L-Alanyl-γ-t-butyl-L-glutamyl-L-asparagine t-butyl ester

A solution of Z-Ala-Glu(OtBu)-Asn-OtBu (40.0 g, 0.069 mol) in MeOH (200 ml) containing 10% Pd/C (5.0 g) was hydrogenated in a Parr hydrogenator until the theoretical amount of H₂ was taken up (and no further uptake of H₂ was observed). The reaction mixture was filtered through celite (topped with a bed of MgS0₄) and evaporated to dryness. A total of 570.9 g of Z-Ala-Glu(OtBu)-Asn-OtBu was reduced and the combined material triturated with ether. The resultant while solid was filtered and dried in vacuo to give 427.6 g (97.5%); mp. 112-118°; 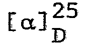 -18.96° (c 1, MeOH); R_{f} 0.15 (CHC1_{3/}MeOH/AcOH; 80/20/5).

### (e) N-Benzyloxycarbonyl-γ-t-butyl-L-qlutamyl-L-alanyl-γ-t-butyl-L-glutamyl-L-asparaqine t-butyl ester

A solution of Z-Glu(OtBu)-OH (269.9 g, 0.80 mol, 1.04 eq.) in DMF (500 ml) was placed in a 3-1 3-necked round bottom flask fitted with a thermometer, mechanical stirrer, dropping funnel and immersed in a dry ice-acetone bath at -15°. The reaction was carried out under an atmosphere of N₂ᵣ cooled to -15° and precooled (-20°) N-methylmorpholine (125 g, 1.24 mol, 1.6 eq.) was added. After stirring for 4 min. at -15°, the reaction mixture was cooled to -35° and precdaled (-20°) isobutylchloroformate (109.3 g, 0.80 mol, 1.04 eq.) was added portionwise over a 3 min. period (temperature must not exceed -15°). The reaction mixture was stirred for an additional 4 min. at -15°, cooled to -35° and a precooled (-20°) solution of H-Ala-Glu(OtBu)-Asn-OtBu (341.4 g, 0.768 mol, 1 eq.) in DMF (750 ml) was added as rapidly as possible while maintaining -15° to -20°. After the addition was complete the reaction mixture was stirred at -15° for 30 min. and at 25° for 2.5 h. The reaction mixture was slowly poured into H₂O (9.5 1) while stirring rapidly and stirred for 2 h. The product was collected by filtration and washed with H₂0 (until the filtrate was free of Cl ) and dried in vacuo. A total of 558.7 g was obtained. Recrystallization from CH₃CN (refluxing required) gave 525.5 g (89.6%) of white solid; mp. 212-214°; 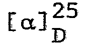 -15.82° (c 1, DMF); R_{f} 0.83 (CHC1₃/MeOH/ AcOH; 85/10/5).

### (f) γ-t-Butyl-L-glutamyl-L-alanyl-γ-t-butyl-L-glutamyl-L-asparagine t-butyl ester (I)

A suspension of Z-Glu(OtBu)-Ala-Glu(OtBu)-Asn-OtBu (147.3 g, 0.193 mol) in MeOH (1.6 1) containing 5% Pd/BaSO₄ (34.1 g) was hydrogenated in a Vibromixer apparatus for 3 h. The reaction mixture was filtered through celite and evaporated to 150 ml. Ether (1.4 1) was added and the resultant white solid was filtered and dried in vacuo. Yield: 103.1 g; second crop, 15.0 g (97.2%); mp. 161-164°; 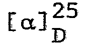 -29.55° (c 1, MeOH); R_{f} 0.70 (n-BuOH/AcOH/EtOAc/H₂0; 1/1/1/1); R_{f} 0.26 (CHCl_{3/}MeoH/AcOH; 80/20/5).

### Example 2

### Synthesis of Fragment II (N-Benzyloxycarbonyl-y-t-butyl-L-glutamyl-L-valyl-L-valyl-γ-t-butyl-L-glutamic acid

### (a) N-Benzyloxycarbonyl-L-valyl-γ-t-butyl-L-glutamic acid methyl ester

N₋Benzyloxycarbonyl-L-valine (144 g, 0.58 mol, 1.2 eq) was dissolved in 400 ml freshly distilled THF and stirred mechanically in a 3-1 3-necked round bottom flask fitted with a thermometer and dropping funnel and immersed in a dry ice-alcohol bath at -15°. While stirring at -15°, N-methylmorpholine (55 ml, 0.58 mol, 1.2 eq) was added dropwise. The temperature was maintained at -15° and isobutylchloroformate (66.2 ml, 0.58 mol, 1.2 eq) was added dropwise over a 2 min. period. The reaction mixture was stirred for an additional 2 min. at -15° and a precooled (-20°) solution of L-glutamic acid a-methyl ester y-t-butyl ester·HCl (120.8 g, 0.48 mol) in 300 ml THF and 150 ml DMF was added dropwise with simultaneous addition of N-methylmorpholine (46 ml, 0.48 mol). The addition took 6 min. while maintaining the temperature below -15°. The reaction mixture was stirred for 30 min. at -15°, and for 3 h at 25°, evaporated in vacuo and the residue was dissolved in EtOAc (900 ml) and washed with 10% NaHCO₃ (3 x 200 ml), 1M citric acid (3 x 200 ml) and saturated NaCl solution (1 x 200 ml). The aqueous washes were backwashed with EtOAc and the combined organic layers were dried over MgSO₄, filtered and concentrated to ~ 200 ml and petroleum ether (1 1) was added. The solid was collected and dried in vacuo, a second crop was obtained from the mother liquor, and the combined crops were recrystallized from CC1₄ (400 ml)/petroleum ether (1.2 1) to yield 168.5 g (78%) of white crystalline product; mp. 66.5-68°; R_{f} 0.74 (n-BuOH/AcOH/EtOAc/H20; 2/1/1/1); R_{f} 0.73 (CHCl₃/CH₃OH/ AcOH; 80/5/1); 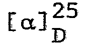 -28.91, (c 1, MeOH).

### (b) N-Benzyloxycarbonyl-L-valyl-L-valyl-y-tert.butyl-L-glutamic acid a-methyl ester

(A) N-Benzyloxycarbonyl-L-valyl-γ-tert.butyl-L-glutamic acid methyl ester (58.0 g, 128.8 mmol) was dissolved in DMF/THF (50 ml/160 ml), 5% P'd/BaSO₄ (12.8 g) was added and the mixture was hydrogenated in a Vibromixer apparatus for 2.5 H. The reaction mixture was filtered through celite, cooled to 0° and the resultant solution of L-valyl-y-tert. butyl-L-glutamic acid methyl ester was placed in the freezer until used in the coupling step below.

(B) N-Benzyloxycarbonyl-L-valine (38.8 g, 154.6 mmol, 1.2 eq) was dissolved in 45 ml freshly distilled THF in a 2-1 3-necked round bottom flask fitted with a thermometer, mechanical stirrer and dropping funnel and immersed in a dry ice-alcohol bath at -15°. While stirring at -15°, N-methylmorpholine (17.3 ml, 154.6 mmol) was added. This was followed by the addition of isobutylchloroformate (20.3 ml, 154.6 mmol) dropwise over a 2 min. period. The reaction mixture was stirred an additional 2 min. at -15° and the precooled (-20°) solution of L-valyl-γ-tert.butyl-L-glutamic acid α-methyl ester (128.8 mmol) from above was added over a 2.7 min. period. The reaction mixture was stirred at -15° for 30 min. and at 25° for 17 h, evaporated in vacuo and the residue was dissolved in CHCl₃ (600 ml) and washed with 10% NaHCO, (3 x 300 ml), saturated NaCl (1 x 300 ml), 1M citric acid (2 x 300 ml) and saturated NaCl (1 x 300 ml). The aqueous phases were back-washed with CHC1₃ and the combined organic layers were dried over MgS0₄, filtered and evaporated in vacuo. The product was recrystallized from i-propanol (4 1) to yield 41.6 g (59%) of white crystalline product; mp. 203-204°; R_{f} 0.47 (CHCl₃/CH₃OH/AcOH: 80/2/0.4); R_{f} 0.74 (CHCl₃/CH₃OH/AcOH; 80/5/1); 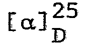 -7.40° (c 1, DM_{F}).

### (c) L-Valyl-L-valyl-y-tert.butyl-L-glutamic acid a-methyl ester

N-Benzyloxycarbonyl-L-valyl-L-valyl-y-tert.butyl-L-glutamic acid a-methyl ester (19.5 g, 35.5 mmol) was dissolved in 250 ml DMF, 5% Pd/BaSO₄ (6 g) was added and the mixture was hydrogenated in a Vibromixer apparatus for 3 h. The reaction mixture was filtered through celite, washed with 50 ml DMF, and evaporated in vacuo to yield 14.7 g (100%) of a white solid; mp. 126-127°; R_{f} 0.35 (CHC1₃/CH₃0H/AcOH; 80/10/5); 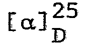 -41.05° (c 1, CH₃0H).

### (d) N-Benzyloxycarbonyl-y-t-butyl-L-glutamyl-L-valyl-L-valyl-y-t-butyl-L-glutamic acid a-methyl ester

Z-Glu(OtBu)-OH (14.4 g, 42.6 mmol, 1.2 eq) was dissolved in THF (120 ml) and placed in a 1-1, 3-necked round bottom flask fitted with a thermometer, mechanical stirrer and immersed in a dry ice-alcohol bath at -15°. N-Methylmorpholine (4.5 ml, 42.6 mmol, 1.2 eq) was added while stirring at -15° and isobutylchloroformate (5.4 ml, 42.6 mmol, 1.2 eq) was added dropwise over a 2 min. period. The reaction mixture was stirred for 2 min. at -15° and a precooled (0°) solution of H-Val-Val-Glu(OtBu)-OMe (14.7 g, 35.5 mmol) in THF/DMF (100 ml/20 ml) was added dropwise over a 3 min. period maintaining the temperature at -15°. Stirring proceeded at -15° for 30 min. and at 25° for 18 h. The reaction mixture was evaporated to dryness, the white solid was triturated with i-propanol and filtered. A second crop of product was obtained from the mother liquor. The total amount of product was 23 g (88%); mp. 210₋211°, R_{f} 0.58 (CHCl₃/CH₃OH/AcOH; 80/2/0.4); 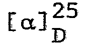 -22.53° (c 1, DMSO).

### (e) N-Benzyloxycarbonyl-y-t-butyl-L-glutamyl-L-valyl-L-valyl-γ-t-butyl-L-glutamic acid (II)

A solution of Z-Glu(OtBu)-Val-Val-Glu(OtBu)-OMe (46.5 g, 63.3 mmol) in DMF (550 ml) was treated with 1N NaOH (79.2 ml, 79.2 mmol, 1.25 eq) and stirred magnetically for 3 h at 25°. It was cooled in an ice-bath and 1N HC1 (190 ml) was added followed by 1.25 1 H₂0. The suspension was stirred for 2 h at 25° and the product collected by filtration, washed with 1 1 H₂0 and dried in vacuo. Yield: 41.9 g (91.8%). An analytical sample was recrystallized from Me4H/H₂O; mp. 193-196°; 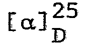 -51.66° (c 1, MeOH); R_{f} 0.34 (CHC13/MeOH/AcOH; 80/5/1).

### Example 3

### Synthesis of Fragment III (N-Benzyloxycarbonyl-y-t-butyl-L-glutamyl-N^{ε}-t-butyloxycarbonyl-L-lysyl-N^{ε}-t-butyloxycarbonyl-L-lysine)

### (a) N-Benzyloxycarbonyl-N -tert-butyloxycarbonyl-L-lysine N-hydroxysuccinimide ester

Z-Lys(Boc)-OH·DCHA (454 g; 0.808 mol) was stirred magnetically in ethyl acetate/0.5N H₂SO₄ (1.5 1/2 1) until all of the white solid was in solution. The organic layer was collected, dried over MgSO₄, filtered and evaporated. The resultant free acid was placed in a 3-1, 3-necked round bottom flask fitted with a thermometer, drying tube and mechanical stirrer and stirred at 0° in ethyl acetate/ dioxane (400 ml/1000 ml). N-Hydroxysuccinimide(93.0 g; 0.808 mol) and DCC (166.6 g; 0.808 mol) were added and the reaction stirred at 0° for 1 h and at 25° for 16 h. The reaction mixture was filtered and the filtrate was evaporated in vacuo. The oily residue was dissolved in i-propanol (500 ml), petroleum ether (~700 ml) was added, the mixture was cooled to 4° for several hours and then filtered to yield 350.4 g (91%) of solid product: mp. 95-97°; R_{f} 0.81 (CHCl₃/CH₃OH/AcOH; 80/5/1); 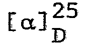 -17.76° (c 1, dioxane).

### (b) N^{α}-Benzyloxycarbonyl-N^{ε}-t-butyloxycarbonyl-L-lysyl- _{N}^{E-}t-butyloxycarbonyl-L-lysine

A solution of Z-Lys(Boc)-OSu (239 g; 0.5 mol) in DMF (1.5 1) was stirred at 0° in a 3-1, 3-necked round bottom flask fitted with a mechanical stirrer and thermometer. H-_{L}ys(Boc)-OH (123 g; 0.5 mol) was added followed by Et₃N (70 ml; 0.5 mol). The reaction mixture was stirred at 0° for 30 min. and at 25° for 18 h and evaporated in vacuo. The residue was dissolved in Et_{O}Ac (1.5 1) and was washed with 1M citric acid (2 x 500 ml) and saturated NaCl solution (500 ml), dried over MgSO₄, filtered and dicyclohexylamine (100 ml) was added. After standing in the cold room for several hours, the white salt was collected and dried to yield 379.8 g (96.4%); mp 114-118°. The DCHA salt Z-Lys(Boc)-Lys(Boc)-OH·DCHA (200 g; 0.25 mol) was stirred in ether/0.5N H₂SO₄ (800 ml each) until all the solid was dissolved. The ether phase was extracted, dried over MgSO₄, filtered and evaporated in vacuo to an oil which was used directly in the next step. 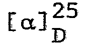 3.06° (c 1, _{M}eOH); R_{f} 0.52 (CHC1_{3/}MeOH/AcOH; 80/5/1).

### (c) N^{ε}-t-Butyloxycarbonyl-L-lysyl-N^{ε}-t-butyloxycarbonyl-L-lysine

The oily product from the preceding step (0.25 mol) was dissolved in CH₃0H (1.2 1) and hydrogenated with 5% Pd/BaS0₄ (30 g) for 4 h in a Vibromixer apparatus. The reaction mixture was filtered through celite and evaporated to dryness to yield 102.5 g (86.4%) of white solid; mp. 189-190°; 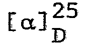 +18.19° (c 1, CH₃OH); R_{f} 0.73 (n-BuOH/ AcOH/EtOAc/H20; 1/1/1/1).

### (d) N-Benzyloxycarbonyl-γ-t-butyl-L-qlutamic acid N-hydroxysuccinimide ester

Z-Glu(OtBu)-OH (168.5 g; 0.5 mol) was dissolved in freshly distilled THF (1 1) and stirred at 0° in a 2-1, 3-necked round bottom flask fitted with a drying tube, mechanical stirrer and thermometer. While stirring at 0°, HOSu (63.6 g; 0.55 mol) and DCC (114 g; 0.55 mol) were added. The reaction mixture was stirred for 1 h at 0° and 18 h at 25°. At, the end of this time the reaction mixture was filtered and the filtrate was evaporated to dryness. The white solid was recrystallized from i-propanol to yield 195.9 g (90.3%); mp. 105-106°; R_{f} 0.66 (CHCl₃/CH₂OH/AcOH; 80/5/1); 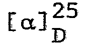 -31.8° (c 1.8, _{E}tOH).

### (e) N-Benzyloxycarbonyl-γ-t-butyl-L-glutamyl-N^{ε}-t-butyl- oxyoarbonyl-L-lysyl-N -t-butyloxycarbonyl-L-lysine (III)

A solution of Z-Glu(OtBu)-OSu (95.5 g; 0.22 mol; 1.1 eq.) in DMF (400 ml) was stirred at 0° in a 2-1, 3-necked round bottom flask fitted with a mechanical stirrer, thermometer and drying tube. H-Lys(Boc)-Lys(Boc)-OH (96.5 g; 0.2 mol) was added as a solid, portionwise, along with Et₃N (30.7 ml; 0.2 mol) and the pH of the reaction mixture was adjusted to 8 with excess Et₃H [DMF (200 ml) was used to wash in all of the H-Lys(Boc)-Lys(Boc)-OH]. The reaction mixture was stirred at 0° for 1 h and at 25° for 18 h, then evaporated in vacuo. The residue was taken up in EtOAc (900 ml) and washed with 1M citric acid (2 x 400 ml) and saturated NaCl (600 ml), dried over MgS0₄, filtered and evaporated to half the volume. Ether (500 ml) was added and after cooling a white solid was collected to give 139.8 g (88%); mp. 150-152°; R_{f} 0.83 (CHCl₃/CH₃OH/AcOH; 80/10/5); R_{f} 0.49 (CHCl₃/CH₃OH/AcOH; 80/5/1); 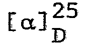 -13.06 (c 1, CH₃0H).

### Example 4

### Synthesis of Fragment IV (N-Benzyloxycarbonyl-β-t-butyi-L-aspartyl-L-leucyl-N^{ε}-t-butyloxycarbonyl-L-lysine)

### (a) N-Benzyloxyaarbonyl-L-leucine-N-hydroxysuccinimid ester

Z-Leu-OH·DCHA (178.65 g; 0.4 mol) was stirred magnetically in ethyl acetate/0.5N H₂5O₄ (1 1/1 1) until all of the white solid was in solution. The organic layer was collected, washed with 0.5N H₂S0₄ (3 x 300 ml) and distilled water (3x 300 ml). The aqueous layers were combined and extracted with ethyl acetate. The organic layers were combined, dried (MgSO₄), filtered and evaporated. The resultant free acid (106.1 g; 0.4 mol) was dissolved in CH₂C1₂ (300 ml) and placed in a 2 1, 3-necked round bottom flask fitted with a thermometer, drying tube and mechanical stirrer. N-Hydroxysuccinimide (50.63 g; 0.44 mol) and THF (100 ml) were added and the mixture was stirred. The resultant solution was cooled (ice-bath), DCC (90.78 g; 0.44 mol) was added and the mixture was stirred at 0° for 1 h and 25° for 3.5 h. The reaction mixture was filtered and the filtrate evaporated in vacuo. The residue was dissolved in CH₂C1₂ (450 ml), filtered (celite), hexane was added to turbidity (1.1 1) and the mixture was stored at 4° for 12 h. The crystals were filtered and dried yielding 122.8 g (84.7%) of product: mp. 115-117°; 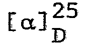 -32.35° (c 1, dioxane).

### (b) N-Benzyloxycarbonyl-L-leucyl-N^{ε}-t-butyloxycarbonyl-L-lysine

A solution of Z-Leu-OSu (122.5 g, 0.338 mol) in DMF (1.2 1) was stirred at 0° in a 3 1, 3-necked round bottom flask fitted with a mechanical stirrer and thermometer. H-L^{y}s(Boc)-OH (83.26 g, 0.338 mol) was added and the solution stirred at 0° for 15 min. Triethylamine (47.6 ml, 0.338 mol) was added and the reaction mixture was stirred at 0° for 30 min. and at 25° for 3 h. Additional triethylamine (20 ml) was added to maintain the pH at 8 and the reaction mixture was stirred for an additional 20 h. At the end of this time, TLC showed some unreacted H-Lys(Boc)-OH and the reaction mixture was stirred for an additional 24 h (pH 8). The reaction mixture was cooled (0°), acidified with glacial acetic acid (160 ml) and evaporated in vacuo. The residue (oil) was dissolved in EtOAc (1.5 1) and washed with 1M citric acid (2 x 600 ml) and 10% NaCl (2 x 700 ml), dried (MgSO₄), filtered and concentrated in vacuo. The oily residue was dissolved in anhydrous ether (4 1) and dicyclohexylamine (70.7 ml) was added followed by the addition of 1 1 of ether. After standing in the cold room for 72 h, the white salt was collected and dried to yield 210.8 g (90%); mp. 125-128° (recrystallization of an analytical sample increased the mp. to 143-146°)_{;} 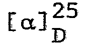 -4.17° (c 1, _{M}eOH); R 0.39 (CHCl₃/MeOH/AcOH: 85/10/5). The free acid was generated from the DCHA salt in the following manner: Z-Leu-Lys(Boc)-OH·DCHA (201.9 g, 0.291 mol) was stirred in EtOAc/0.5N H₂SO₄ (2 1/727 ml) until all of the solid was dissolved. The organic layer was separated, washed with 0.5N H₂SO₄ (730 ml), dried (MgSO₄) and evaporated in vacuo. Yield: 150.6 g (100%); 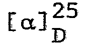 +3.38° (c 1, CHCl₃); R_{f} 0.64 (CHCl₃/MeOH/AcOH): 85/10/5).

### (c) L-Leucyl-N^{ε}-tert-butyloxycarbonyl-L-lysine

A solution of Z-Leu-Lys(Boc)-OH (41.5 g, 0.084 mol) in methanol (210 ml) was hydrogenated in a Vibromixer apparatus with 10% Pd/C (5.0 g) for 3.5 h. The precipitated product dissolved on addition of H₂0 (120 ml). The catalyst was removed by filtration (celite), washed twice with H₂0 and the combined filtrates were evaporated to near dryness. The residue was reevaporated from MeOH (2x) and Et₂0 (2x). The solid product was collected, washed with Et₂0 and dried to afford 26.9 g of white solid. Recrystallization from warm i-propanol (420 ml) and MeOH (350 ml) afforded, after drying, 24.6 g of white crystalline product (81.5%); mp 142-143°; R_{f} 0.63 (n-BuOH/AcOH/EtOAc/H20; 1/1/1/1); 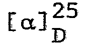 +20.76° (c 1, MeOH).

### (d) N-Benzyloxycarbonyl-ß-tert-butyl-L-aspartic acid N-hydroxysuccinimide ester

Z-Asp(OtBu)-OH·DCHA (378 g; 0.750 mol) was stirred magnetically at 0° in a mixture of ethyl acetate/0.5N H₂SO₄ (3.0 1/1.875 1) until all of the white solid was in solution. The organic layer was collected, washed with ice-cold 0.5N H₂SO₄ (1.8 1) and water (3 x 1 1), dried (MgSO₄), filtered and evaporated in vacuo. Yield: 252 g (100%); R 0.63 (CHCl₃/MeOH/AcOH; 85/10/5); 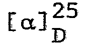 -10.10° (c 1, pyridine). The resultant free acid (252 g; 0.75 mol) and N-hydroxysuccinimide (94.95 g; 0.825 mol) were placed in a 12 1, 3-necked round bottom flask fitted with a thermometer, drying tube, mechanical stirrer and stirred at 25° in THF (4.2 1). After all of the solids had dissolved, the reaction mixture was cooled (0°), DCC (170.2 g; 0.825 mol) and THF (0.75 1) were added and the mixture was stirred at 0° for 1 h and 25° for 5 h. The reaction mixture was filtered and the filtrate evaporated in vacuo. The residue was dissolved in CH₂C1₂ (2 1) and stored (0°). The solution was filtered and the filtrate evaporated in vacuo. The residue was dissolved in CH₂C1₂ (warm)/hexane (1 1/ 4 1) and stored (0°). The resultant crystals were washed with hexane/CH₂Cl₂ (9:1, 1 1) and hexane (1 1) and dried. Yield: 274 g (86.5%); mp. 150-151°; [α] ²⁵_{D} -26.36° (c 1, DMF).

### (e) N-Benzyloxycarbonyl-ß-tert-butyl-L-aspartyl-L-leucyl-N^{E-}tert-butyloxycarbonyl-L-lysine (IV)

A solution of Z-Asp(OtBu)-OSu (115.6 g; 0.275 mol) in DMF (1.16 1) was stirred at 0° in a 3 1 3-necked round bottom flask fitted with a mechanical stirrer and thermometer. H-Leu-Lys(Boc)-OH (89.86 g; 0.250 mol) was added followed by Et₃N (35.2 ml; 0.25 mol). The reaction mixture was stirred at 0° for 0.5 h and at 25° for 1.5 h. Additional Et₃N (17 ml) was added (to maintain pH 8) and the reaction mixture was stirred at 25° for 20 h. The reaction mixture was evaporated, dissolved in EtOAc (3 1), washed with 1M citric acid (3 x 1 1), H₂0 (3 x 1 1), dried (MgSO₄) and evaporated in vacuo. The residual paste was dissolved in anhydrous ether (5 1) and stirred magnetically while DCHA (53.5 ml; 0.268 mol) was added slowly. After standing in the cold room (20 h) the white salt was collected and washed with anhydrous ether (2 x 0.5 1) and petroleum ether (2 x 0.5 1). Yield: 177 g (84%); mp. 173-174°;_{;} 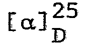 -12.57° (c 1, MeOH). Z-Asp(OtBu)-Leu-Lys(Boc)-OH·DCHA (198 g; 0.234 mol) was stirred at 0° in a mixture of EtOAc/0.5N H₂SO₄ (3.2 1/1.19 1) until all of the solid was dissolved. The organic layer was collected, washed with H₂0 (4 x 1 1), dried (MgSO₄), filtered and evaporated in vacuo. The residue was triturated with petroleum ether and dried. Yield: 165.5 g (100%); 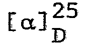 -1.16° (c 1, CHCL₃); R_{f} 0.73 (CHC1_{3/}MeOH/AcOH; 85/10/5).

### Example 5

### Synthesis of Fragment V (Z-lle-Thr(tBu)-Thr(tBu)-Lys(Boc)-OH)

### (a) N^{ε}-t-Butyloxycarbonyl-L-lysine phenyl ester tosylate

Z-Lys(Boc)-OPh (143 g, 313 mmol) and TOS-OH·H_{æ}O (59.54 g, 313 mmol) were dissolved in 1 1 DMF and hydrogenated in the presence of 10% Pd/C. The solution was filtered and concentrated in vacuo to about 500 ml. Crystallization was achieved by the addition of ethyl acetate (5 1). Yield: 136 g (87%); mp. 163-165°; 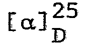 +8.3° (c = 1, DMF).

### (b) N-Benzyloxycarbonyl-O-t-butyl-L-threonyl-N^{ε}-t-butyloxycarbonyl-L-lysine phenyl ester

Z-Thr(tBu)-OH (180.35 g, 583 mmol) was dissolved in 850 ml DMF and cooled to -35°. While stirring at -35°, N-methylmorpholine (64.2 ml, 583 mmol) was added, followed by the addition of isobutylchloroformate (76.2 ml, 583 mmol). The reaction mixture was activated for 2 min. at -15° and quickly cooled to -30°. A precooled solution (-25°) of H-Lys(Boc)-OPh-Tos-OH (262.1 g, 530 mmol) in 800 ml DMF and N-methylmorpholine (58.4 ml, 530 mmol) was added simultaneously. The reaction mixture was stirred for 15 min. at -15° and 1 h at 25° and evaporated in vacuo. The residue was dissolved in 3 1 ethyl acetate and washed with 10% Na₂CO₃ solution (3 x 1.5 1), saturated NaCl solution (4 x 1 1), 5% KHS04/10% K₂SO₄ solution (3 x 1.5 1), and saturated NaCl solution, dried over Na₂SO₄, filtered and evaporated in vacuo. The residue was crystallized from ether (1 1)/hexane (3 1) or from isopropyl acetate (1.5 1)/ hexane (4 1). Yield: 258.4 g (79.5%); mp. 72°; 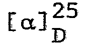 +11.8° (c = 1, CHC1₃). When the material was crystallized from isopropyl acetate/hexane a melting point of 106° resulted.

### (c) N-Benzyloxycarbonyl-O-t-butyl-L-threonyl-O-t-butyl-L-threonyl-N -t-butyloxycarbonyl-L-lysine phenyl ester

Z-Thr(tBu)-Lys(Boc)-OPh (251.6 g, 410 mmol) dissolved in 1 1 DMF was hydrogenated in the presence of Tos-OH·H₂O (78 g, 410 mmol) and 10% Pd/C. After the hydrogenation was completed the catalyst was filtered off.

Z-Thr(tBu)-OH (139.2 g, 450 mmol) was dissolved in 700 ml DMF and cooled to -35°. While stirring at -35°, N-methylmorpholine (49.6 ml, 450 mmol) was added, followed by the addition of isobutylchloroformate (58.8 ml, 450 mmol). The reaction mixture was activated for 2 min. at -15° and quickly cooled to -30°. The precooled (-25°) DMF solution from the hydrogenation step and N-methylmorpholine (45.2 ml, 410 mmol) were added simultaneously. The reaction mixture was stirred for 15 min. at -15° and 1 h at 25° and evaporated in vacuo. The residue was dissolved in 2 1 ethyl acetate and washed with 10% Ha₂CO₃ solution (3 x 1.5 1), saturated NaCl solution (1 x 1 1), 5% KHS0_{4/}10% K₂SO₄ solution (3 x 1.5 1), and saturated NaCl solution, dried over Na₂SO₄, filtered and evaporated in vacuo. The residue was crystallized from ether (1 1)/hexane (4 1). Yield: 240.2 g (76%); mp. 124°; 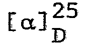 +26.4° (c = 1, CHCl₃).

### (d) N-Benzyloxycarbonyl-L-isoleucyl-O-t-butyl-L-threonyl-O-t-butyl-L-threonyl-N^{ε}-t-butyloxycarbonyl-L-lysine phenyl ester

A solution of Z-Thr(tBu)-Thr(tBu)-Lys(Boc)-OPh (346.9 g, 450 mmol) in 1 1 DMF was hydrogenated in the presence of Tos-OH·H₂O (85.6 g, 450 mmol) and 10% Pd/C. After the hydrogenation was completed the catalyst was filtered off.

Z-Ile-OH (132.66 g, 500 mmol) was dissolved in 700 ml DMF and cooled to -35°.While stirring at -35°, N-methylmorpholine (55 ml, 500 mmol) was added, followed by the addition of isobutylchloroformate (65 ml, 500 mmol). The reaction mixture was activated for 5 min. at -15° and quickly cooled to -20°..The precooled DMF solution (-25°) from the hydrogenation step and N-methylmorpholine (50 ml, 450 mmol) were added simultaneously. The reaction mixture was stirred for 15 min. at -15° and 1 h at 25° and evaporated in vacuo. The residue was dissolved in 3 1 ethyl acetate and washed with 10% Na₂CO₃ solution (2 x 1.5 1), saturated NaCl solution (1 x 1 1), 5% KHSO₄/10% K₂SO₄ solution (2 x 1.5 1) and saturated NaCl solution, dried over Na₂SO₄, filtered and evaporated in vacuo. The residue was crystallized from ethyl acetate (2 1)/ether (2 1). Yield: 296 g (74.5%); mp. 150°; 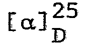 +24.2° (c = 1, CHCl₃),

### (e) N-Benzyloxycarbonyl-L-isoleucyl-o-t-butyl-L-threonyl-O-t-butl-L-threonyl-N^{ε}-t-butyloxycarbonyl-L-lysine, V

Z-Ile-Thr(tBu)-Thr(tBu)-Lys(Boc)-OPh (0.884 g, 1.0 mmol) was dissolved in 25 ml acetone, 4 ml H₂0 were added followed by the addition of 3% H₂0₂ (1.16 ml, 1.0 mmol) and the solution was stirred magnetically. The pH was adjusted to, and maintained at, 10.5 by the addition of O.lN NaOH. After stirring at pH 10.5 for 1.5 h, 0.1N HC1 was added until the reaction mixture reached pH 3. The white solid which formed was collected by filtration, washed with H₂0 (~60 ml) and dried in vacuo to yield 0.781 g (96.6%); mp. 179-180°, Rf 0.63 (CHC13/CH30H/AcOH; 80/5/1); 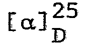 +13.66 (c 1, CH₃0H).

### Example 6

### Synthesis of Fragment VI (H-Ala-Val-Asp(OtBu)-Thr(tBu)-Ser(tBu)-Ser(tBu)-Glu(OtBu)-OPh

### (a) N-Benzyloxycarbonyl-γ-t-butyl-L-qlutamic acid phenyl ester

Z-Glu(OtBu)-OH (202.4 g, 0.6 mol) was dissolved in 1.5 1 ethyl acetate and cooled to 0°. To the stirred solution pyridine (48.4 ml, 0.6 mol), phenol (56.5 g, 0.6 mol) and a precooled solution of DCCI (123.8 g, 0.6 mol) in about 300 ml ethyl acetate were added. The reaction mixture was stirred for 2 h at 0° and for 20 h at room temperature. The reaction mixture was stirred for another 30 min. after the addition of acetic acid (2 ml). Dicyclohexylurea was filtered off and the filtrate was washed with 10% NaHCO₃ solution, saturated NaCl solution, 5% KHSO₄/10% K₂S0₄ solution and saturated NaCl solution and dried over Na₂SO₄. The filtrate was concentrated and crystallized by adding petroleum ether. Yield: 206 g (83%); mp. 74°; [a]_{D} -27° (c = 1, DMF).

### (b) N-Benzyloxycarbonyl-O-t-butyl-L-seryl-γ-t-butyl-L-glutamic acid phenyl ester

A solution of Z-Glu(OtBu}-OPh (198.4 g, 480, mmol) in 1 1 DMF was hydrogenated in the presence of Tos-OH·H₂O (91.3 g, 480 mmol) and 10% Pd/C (20 g). After the hydrogenation was completed the catalyst was filtered off.

Z-Ser(tBu)-OH (147.7 g, 500 mmol) was dissolved in 1.5 1 DMF and cooled to -25°. N-Methylmorpholine (55.1 ml, 500 mmol) and isobutylchloroformate (65.35 ml, 500 mmol) were added and the reaction mixture was activated at -15° for 2 min. The pre-cooled DMF solution (-25°) from the hydrogenation step and N-methylmorpholine (52.9 ml, 480 mmol) were added simultaneously. The reaction mixture was stirred for 15 min. at -15° and 1 h at 25° and evaporated in vacuo. The residue was dissolved in 4 1 of ethyl acetate, extracted with 10% NaHCO₃ solution, 5% KHS0₄/10% K₂SO₄ solution and saturated NaCl solution, dried over Na₂SO₄, filtered and evaporated. The residue was crystallized from ether/hexane. Yield: 223 g (83.5%), mp. 97°; 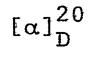 +16.2° (_{c} = 1, CHCl₃).

### (c) N-Benzyloxycarbonyl-O-t-butyl-L-seryl-O-butyl-L-seryl- y-t-butyl-L-qlutamic acid phenyl ester

A solution of Z-Ser(tBu)-Glu(OtBu)-OPh (256.1 g, 460 mmol) in 1.2 1 DMF was hydrogenated in the presence of Tos-oH·H₂O (87.5 g, 460 mmol) and 10% Pd/C ( 25 g). After the hydrogenation was completed the catalyst was filtered off.

Z-Ser(tBu)-OH (145.6 g, 493 mmol) was dissolved in 1.4 1 DMF and cooled to -25°. N-Methylmorpholine (54.3 ml, 493 mmol) and isobutylchloroformate (64.4 ml, 493 mmol) were added to this solution and the mixture was activated at -15° for 2 min. The precooled DMF solution from the hydrogenation step and N-methylmorpholine (50.7 ml, 460 mmol) were added simultaneously. The reaction mixture was stirred for 15 min. at -15° and 1 h at 25° and evaporated in vacuo. The residue was dissolved in 4 1 ethyl acetate and extracted with 10% NaHCO₃ solution, 5% KHS0₄/10% K₂SO₄ solution, and saturated NaCl solution, dried over Na₂SO₄, filtered and evaporated. The residue was crystallized from ethyl acetate/hexane. Yield: 262 g (81.4%); mp. 130-131°; 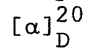 +21.6° (c = 1, CHCl₃). From the motherliquor another crop of 26 g (8%) was obtained. Total yield: 288 g (89.5%).

### (d) N-Benzyloxycarbonyl-O-t-butyl-L-threonyl-O-t-butyl-L-seryl-O-t-butyl-L-seryl-γ-t-butyl-L-ctlutamic acid phenyl ester

A solution of Z-Ser(tBu)-Ser(tBu)-Glu(OtBu)-OPh (270 g, 386 mmol) in 1.2 1 DMF was hydrogenated in .the presence of Tos-OH·H₂O (73.4 g, 386 mmol) and 10% Pd/C (30 g). After the hydrogenation was completed the catalyst was filtered off.

Z-Thr(tBu)-OH (119.4 g, 386 mmol) was dissolved in 1.2 1 DMF and cooled to -25°. N-Methylmorpholine (42.5 ml, 386 mmol) and isobutylchloroformate (50.5 ml, 386 mmol) were added to this solution and the mixture was activated at -15° for 2 min. The precooled DMF solution from the hydrogenation step and N-methylmorpholine (42.5 ml, 386 mmol) were added simultaneously. The reaction mixture was stirred for 15 min. at -15° and 1 h at 25° and evaporated in vacuo. The residue was dissolved in 4 1 ethyl acetate, extracted with 10% NaHCO₃ solution, 5% KHSO₄/10% K₂S0₄ solution, and saturated NaCl solution, dried over Na₂SO₄, filtered and evaporated. The residue was crystallized from ethyl acetate/hexane. Yield: 270.5 g (81.8%); mp. 144-145°; 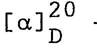 +37.2° (c = 1, CHC1₃). From the motherliquor another crop of 19.6 g (6%) was obtained. Total yield: 290.1 g (87.7%).

### (e) N-Benzyloxycarbonyl-L-valyl-ß-t-butyl-L-aspartic acid

To a stirred suspension of H-Asp(OtBu)-OH (18.9 g, 100 mmol) in 400 ml DMF at 0° triethylamine (13.9 ml, 100 mmol) was added followed by Z-Val-OSu (38.3 g, 110 mmol) and pyridine (8.1 ml, 100 mmol). The reaction mixture was stirred at 0° for 2 h and at 25° for 20 h. The clear reaction mixture was evaporated to dryness and the residue distributed between ethyl acetate and 5% KHSO₄/10% K₂S0₄ solution. The organic layer was washed with 5% KHS0_{4/}10% K₂SO₄ solution, saturated NaCl solution and dried over Na₂SO₄, filtered and evaporated in vacuo. The residue was crystallized and recrystallized from ethyl acetate/hexane. Yield: 35.9 g (85%); mp. 138-139°; 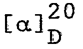 -9.7° (c = 1, _{M}eOH).

### (f) N-Benzyloxycarbonyl-L-alanyl-L-valyl-ß-t-butyl-L-aspartic acid

A solution of Z-Val-Asp(OtBu)-OH (31.7 g, 75 mmol) in 500 ml methanol/25 ml water was hydrogenated in the presence of 10% Pd/C. The hydrogenation mixture was heated, filtered and evaporated in vacuo. The crystalline residue was suspended in 300 ml DMF and cooled to 0°. N-Methylmorpholine (8.25 ml, 75 mmol) was added followed by Z-Ala-OSu (24 g, 75 mmol) and pyridine (6.05 ml, 75 mmol). The reaction mixture was stirred at 0° for 4 h and at 25° for 18 h. The mixture was evaporated in vacuo and the residue was distributed between ethyl acetate and 5% KHSO₄/K₂SO₄ solution. Sufficient water-saturated n-butanol was added to dissolve the crystallizing compound in the organic phase. The organic phase was washed with water (5 x 200 ml) and evaporated in vacuo. The residue was crystallized from ethyl acetate/ isopropyl acetate. Yield: 30.5 g (82.5%); mp. 168°; [α] ²⁰_{D} -37.2° (c = 1, MeOH).

### (g) Z-Ala-Val-Asp(OtBu)-Thr(tBu)-Ser(tBu)-Ser(tBu)-Glu(OtBu)-OPh

A solution of Z-Thr(tBu)-Ser(tBu)-Ser(tBu)-Glu(OtBu)-OPh (200 g, 233 mmol) in 1.2 1 DMF was hydrogenated in the presence of Tos-OH·H₂O (44.4 g, 233 mmol) and 10% Pd/C (20 g). After the hydrogenation was completed the catalyst was filtered off.

Z-Ala-Val-Asp(OtBu)-OH (123.4 g, 250 mmol) was dissolved in 1.5 1 DMF and cooled to -25°. N-Methylmorpholine (27.5 ml,' 250 mmol) and isobutylchloroformate (32.7 ml, 250 mmol) were added to this solution and the mixture was activated for 2 min. at -15°. The precooled DMF solution from the hydrogenation step and N-methylmorpholine (25.7 ml, 233 mmol) were added simultaneously. The reaction mixture was stirred for 15 min. at -15° and 1 h at 25° and poured into 15 1 of water which contained NaHCO₃(23.5 g, 280 mmol). The resulting solid was collected, washed with water and dried in vacuo. This solid was dissolved under warming in chloroform and crystallized by the addition of hexane. Yield: 260 g (93%); mp. 236-237°; 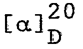 +19.4° (c = 1, CHCl₃).

### (h) H-Ala-Val-Asp(OtBu)-Thr(tBu)-Ser(tBu)-Ser(tBu)-Glu(OtBu)-OC₆H₅, VI

A solution of Z-Ala-Val₋Asp(OtBu)-Thr(tBu)-Ser(tBu)-Ser(tBu)-Glu(OtBu)-OC6H5 (20.16 g, 16.8 mmol) in DMF (400 ml) was hydrogenated with 5% Pd/BaSO₄ (10 g) for 2 h in a Vibromixer apparatus. The reaction mixture was filtered through celite which was washed with additional DMF (228 ml). The combined filtrates were used directly in the next stage of synthesis. A portion of the material was precipitated by addition of H20, filtered and dried in vacuo; mp. >275°; 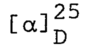 -9.28° (c 1, _{T}F_{E}); R_{f} 0.77 (n-BuOH/AcOH/Pyr/H₂O; 4/7/1/1); R_{f} 0.65 (n-BuOH/H20/AcOH/EtOAc; 1/1/1/1).

### Example 7

### Synthesis of Fragment VII (Ac-Ser(tBu)-Asp(OTßu)-Ala-OSer)

### (a) N-Benzyloxycarbonyl-ß-t-butyl-L-aspartyl-L-alanine benzyl ester

Z-Asp(OtBu)-OH (161.6 g,500 mmol) was dissolved in 750 ml DMF and cooled to -30°.To the stirred solution N-methylmorpholine (55.1 ml, 500 mmol) and isobutylchloroformate (65.4 ml, 500 mmol) were added and the mixture was activated for 2 min. at -15°. A precooled DMF (500 ml) solution of H-Ala-OBzl·Tos-OH (193.3 g, 550 mmol) and N-methylmorpholine (60.6 ml, 550 mmol) were added simultaneously. The reaction mixture was stirred at -15° for 15 min. and at 25° for 1 h, evaporated in vacuo and the residue was taken up in 3.5 1 of ethyl acetate. The organic layer was extracted with 10% Na₂CO₃-solution, water, 5% KHSO₄/10% K₂SO₄ solution and saturated NaCl solution dried over Na₂SO₄, filtered and evaporated in vacuo. Crystallisation was achieved at 0° from ethyl acetate (0.5 1)/hexane (5 1). Yield: 202.9 g (83.8%); mp. 51°; 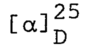 -26.9° (c = 1, MeOH).

### (b) N-Benzyloxycarbonyl-O-t-butyl-L-seryl-ß-t-butyl-L-aspartyl-L-alanine

A solution of Z-Ser(tBu)-OH (29.5 g, 100 mmol) in 300 ml ethyl acetate was cooled to 0°. N-Hydroxysuccinimide (11.5 g, 100 mmol) and DCC (22.7 g, 110 mmol) were added and the reaction mixture was stirred at 0° for 1 h and at 25° for 20 h. The unreacted DCC was destroyed by adding Tos-OH·H₂O (0.2 g) and water (5 ml). After stirring for 1 h at 25° the urea was filtered off and the filtrate was evaporated in vacuo.

Z-Asp(OtBu)-Ala-OBzl (43.6 g, 90 mmol) was dissolved in 1 1 of DMF and hydrogenated in the presence of Tos-OH·H₂O (17.1 g, 90 mmol) and 10% Pd/C. After hydrogenation was completed the catalyst was filtered off. The filtrate was cooled to 0°, neutralized with N-methylmorpholine (9.9 ml, 90 mmol) and added to Z-Ser(tBu)-OSu. Another portion of N-methylmorpholine (9.9 ml, 90 mmol) was added and the reaction mixture was stirred at 0° for 1 h and at 25° for 18 h. 2-Diethylamino-ethylamine (2.83 ml, 20 mmol) was added to the reaction mixture and stirring was continued for 4 h. The solution was evaporated in vacuo, the residue dissolved in 0.5N NaOH (0.5 1) and extracted with ether (3 x 0.2 1). To the alkaline phase 0.4 1 ethyl acetate was added and·5% KHSO₄/10% K₂S0₄ solution. The acidic layer was again extracted with ethyl acetate (2 x 0.3 1). The combined organic layers were washed with 5% KHSO₄/10% K₂S0₄ solution (1 x 0.3 1) and saturated NaCl solution, dried over Na₂SO₄, filtered and evaporated in vacuo. Yield: 37 g (76.5%) of an oil.

### (c) N-Acetyl-O-t-butyl-L-seryl-β-t-butyl-L-aspartyl-L-alanine

The oily product Z-Ser(tBu)-Asp(OtBu)-Ala-OH (16.3 g, 30 mmol) from the preceding step was dissolved in 200 ml methanol and hydrogenated with 10% Pd/C. The catalyst was filtered off and the filtrate was evaporated to dryness. The residue was dissolved in 100 ml DMF, cooled to 0° and N-methylmorpholine (6.6 ml, 60 mmol) as well as acetic anhydride (4.25 ml, 45 mmol) were added under stirring. The reaction mixture was stirred for 15 min. at 25° and evaporated in vacuo. The residue was dissolved in 1N NaOH and extracted with ethyl acetate. The alkaline phase was acidified with 5% KHSO₄/10% K₂S0₄ solution, saturated with K₂S0₄ and serveral times extracted with ethyl acetate. The combined organic layers were washed with saturated NaCl solution, dried over Na₂SO₄ and evaporated to dryness. The residue was crystallized from isopropyl acetate/ether. Yield: 8.4 g (62.7%); mp. 112°; 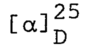 -16.6° (c = 5, MeOH).

### (d) Ac-Ser(tBu)-Asp(OtBu)-Ala-OSu, VII

A solution of Ac-Ser(tBu)-Asp(OtBu)-Ala-OH (24.7 g, 55.4 mmol) in dry THF (526 ml) was treated with HOSu (9.55 g, 83.1 mmol, 1.5 eq.) and cooled to 0°. To the cold mechanically stirred solution DCC (17.14 g, 83.1 mmol, 1.5 eq.) was added portionwise and stirring proceeded at 0° for 30 min. and at 25° for 5 h. The reaction mixture was filtered, the filtrate was evaporated to dryness and the residue crystallized from isopropanol (250 ml). Yield: 23.2 g (77.2%); mp. 155-157°; 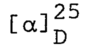 -41.48° (c 1, MeOH); R 0.54 (CHCl₃/MeOH/AcOH; 80/5/1).

### Example 8

### Synthesis of Thymosin a₁ from Fragments I-VII

### (a) Z-Glu(OtBu)-Val-Val-Glu(OtBu)-Glu(OtBu)-Ala-Glu(OtBu)-Asn-OtBu

A solution of Z-Glu(OtBu)-Val-Val-Glu(OtBu)-OH (II, 35.5 g, 0.049 mol) and H-Glu(OtBu)-Ala-Glu(OtBu)-Asn-OtBu (I, 31.5 g 0.05 mol) in DMF (850 ml) was cooled to 0° and HOBt (22.7 g, 0.148 mol, 3 eq.) was added with mechanical stirring. DCC (15.3 g, 0.074 mol, 1.65 eq.) was added and the pH was adjusted to 7.5-8.0 by addition of N-methylmorpholine (~15 ml). After stirring for 1 h at 0° and 23 h at 25° it was filtered and the filtrate was poured into H₂0 (8 1) with additional stirring for 1.5 h. The product was collected by filtration, washed with H₂0 (8 1), dried, washed with MeOH (4 x 250 ml) and dried in vacuo to give 46.8 g. Recrystallization from MeOH gave a white solid. Yield: 39.6 g (60%); mp 244-245°, 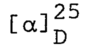 -20.65°(c 0.5, DMSO); R_{f} 0.35 (CHCl₃/MeoH/AcOH; 80/5/1).

### (b) H-Glu(OtBu)-Val-Val-Glu(OtBu)-Glu(OtBu)₋Ala-Glu(OtBu)-Asn-OtBu

A solution of Z-Glu(OtBu)-Val-Val-Glu(OtBu)-Glu(OtBu)-Ala-Glu(OtBu)-Asn-OtBu (42.6 ^{g}, 32.0 mmol) in DMF (650 ml) containing 10% Pd/BaS0₄ (15.0 g) was hydrogenated in a Vibromixer apparatus for 3 h. The reaction mixture was filtered through a bed of celite which was washed with DMF (5 x 250 ml). The combined filtrates were evaporated to600 ml and DMSO (300 ml) added. The resultant solution was used for the next stage of synthesis. A portion was evaporated to dryness, taken up in MeOH and precipitated with Et₂0. The resultant gel was filtered, washed with Et₂0 and dried in vacuo; mp 246-249° (dec.); 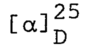 -18.78° (c 1, DMF); R_{f} 0.78 (n-BuOH/AcOH/Pyr/H20; 1/1/1/1).

### (c) Z-Glu(OtBu)-Lys(Boc)-Lys(Boc)-Glu(OtBu)-Val-Val-Glu(OtBu)-Glu(OtBu)-Ala-Glu(OtBu)-Asn-OtBu

The DMF/DMSO-containing solution of H-Glu(OtBu)-Val-Val-Glu(OtBu)-Glu(OtBu)-Ala-Glu(OtBu)-Asn-OtBu (~32 mmol) from (b) was combined with Z-Glu(OtBu)-Lys(Boc)-Lys(Boc)-OH (III, 36.8 g, 46 mmol, 1.44 eq.) and H_{O}Bt (14.7 g, 96 mmol, 3 eq.) and the solution was cooled to 0°. DCC (10.9 g, 52 mmol, 1.65 eq.) was added to the cold mechanically stirred reaction mixture and the pH was maintained at 7.5-8.0 by addition of N-methylmorpholine (21 ml). Stirring proceeded for 2 h at 0° and 21 h at 25°. The resultant gelatinous reaction mixture was poured into H₂0 (9.0 1) and stirred for 4 h. The product was collected by filtration, washed with H₂0 (8 x 500 ml), dried and washed with MeOH using a centrifuge to separate the 2 phases. After removal of the dicyclohexylurea the product was combined and dried in vacuo to give 45.22 g (71.5%); mp. 312° (dec.); 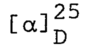 -18.20° (c 1, DMS_{O}); 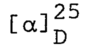 -13.27° (c 1, TFE); R_{f} 0.81 (n-BuOH/ AcOH/Pyr/H₂0; 15/3/10/12); R_{f} 0.52 (CHC1_{3/}MeOH/AcOH; 85/10/5).

### (d) H-Glu(OtBu)-Lys(Boc)-Lys(Boc)-Glu(OtBu)-Val-Val-Glu(OtBu)-Glu(OtBu)-Ala-Glu(OtBu)-Asn-OtBu

A solution of Z-Glu(OtBu)-Lys(Boc)-Lys(Boc)-Glu(OtBu)-Val-Val-Glu(OtBu)-Glu(OtBu)-Ala-Glu(OtBu)-Asn-OtBu (45.0 g, 23 mmol) inTFE (1.11) containing 10% Pd/BaSO₄ (15 g) was hydrogenated in a Vibromixer apparatus for 3 h. The reaction mixture was filtered through celite which was washed with TFE (3 x 100 ml). The combined filtrates were evaporated to dryness and taken up in DMF/DMSO (1300 ml/650 ml) for use in the next stage of synthesis. A portion of the residue was precipitated from TFE-H₂0, filtered and dried in vacuo; mp. 326-327°; 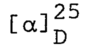 -17.65° (c 1, DMSO); R 0.73 (n-BuOH/AcOH/EtOAc/H20; 1/1/1/1).

### (e) Z-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-OtBu

The DMF/DMSO-containing solution of H-Glu(OtBu)-Lys-(Boc)-Lys(Boc)-Glu(OtBu)-Val-Val-Glu(OtBu)-Glu(OtBu)-Ala-Glu(OtBu)-Asn-OtBu (~23 mmol) from step (d) was combined with Z-Asp(OtBu)-Leu-Lys(Boc)-OH (IV, 18.6 g, 28.0 mmol, 2.1 eq.) and HOBt (10.62 g, 69.0 mmol, 3.0 eq.) and the solution was cooled to 0°. DCC (7.87 g, 38.0 mmol, 1.65 eq.) was added to the cold reaction mixture under mechanical stirring and the pH was maintained at 7.5-8.0 by addition of N-methylmorpholine (~17 ml). Stirring proceeded for 1 h at 0° and 23 h at 25°. The resultant reaction mixture was poured into H₂0 (10 1) and stirred for 1.5 h. The product was collected by filtration, washed with H₂0 (5 1), dried and washed with MeOH using a centrifuge to separate the 2 layers. After removal of the dicyclohexylurea the product was pooled and dried in vacuo to give 44.56 g (77.9%); mp.>330°; 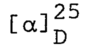 -11.56° (c 1, TFE); R_{f} 0.16 (CHC1_{3/}MeOH/ AcOH; 80/5/1); R_{f} 0.36 (CHCl₃/MeOH/TFE/AcOH; 85/10/2.5/1).

### (f) H-Asp(OtBu)-Leu-Lys(Boc)-Glu(OtBu)-Lys(Boc)-Lys(Bac)-Glu(OtBu)-Val-Val-Glu(OtBu)-Glu(OtBu)-Ala-Glu(OtBu)-Asn-OtBu

A solution of Z-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-OtBu (35.36 g, 14.22 mmol) in TFE (720 ml) containing 5% Pd/Baso₄ (11.8 g) was hydrogenated in a Vibromixer apparatus for 4 h. The reaction mixture was filtered through celite, evaporated nearly to dryness and precipitated with H20 (2 1). The product was collected by filtration and dried in vacuo. Yield: 32.74 g (95.1%); mp. 320°; 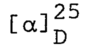 -6.54° (c 1, TFE); R 0.42 (CHC1_{3/}MeOH/ AcOH; 85/10/5).

### (g) Z-Ile-Thr(tBu)-Thr(tBu)-Lys(Boc)-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-OtBu

A solution of H-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-OtBu (16.72 g, 6.95 mmol) in DMF/DMSO (654 ml/327 ml) was combined with Z-Ile-Thr(tBu)-Thr(tBu)-Lys(Boc)-OH (V, 8.76 g, 10.4 mmol, 1.5 eq.) and HOBt (3.19 g, 20.9 mmol, 3 eq.) and the mixture was cooled to 0°. DCC (2.37 g, 11.5 mmol, 1.65 eq.) was added to the cold reaction mixture under mechanical stirring and the pH was maintained at 7.5-8.0 by addition of N-methylmorpholine ( 4.7 ml). Stirring proceeded for 1 h at 0° and 48 h at 25°. The reaction mixture was cooled to 0° and a second coupling was carried out as follows: Z-Ile-Thr(tBu)-Thr(tBu)-Lys(Boc)-OH (V, 2.92 g, 3.47 mmol, 0.5 eq.) HOBt (1.06 g, 6.95 mmol, 1 eq.) and _{DCC} (0.86 g, 4.17 mmol,0.6 eq.) were added under stirring and stirring proceeded at 0° for 1 h and at 25° for 17 h. The reaction mixture was poured into H₂0 (5 1), stirred, filtered and washed with H₂0 (3 x 1 1), MeOH (3 x 500 ml), DMSO (3 x 500 ml) and MeOH (2 x 500 ml). The product was dried in vacuo. Yield: 15.95 g (73.2%); mp. 347-349° (dec.); R_{f} 0.21 (CHC1₃/MeOH/AcOH; 80/5/1); R_{f} 0.56 (CHC1_{3/}MeOH/AcOH; 85/10/5); R_{f} 0.46 (CHC1_{3/}MeOH/ TFE/AcOH; 85/10/2.5/1); 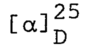 -9.17° (c 0.1, TFE).

### (h) H-Ile-Thr(tBu)-Thr(tBul-Lys(Soc)-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-OtBu

A solution of Z-Ile-Thr(tBu)-Thr(tBu)-Lys(Boc)-Asp-(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-OtBu (32.24 g, 10.25 mmol) in TFE (700 ml) was hydrogenated in a Vibromixer apparatus with 10% Pd/C (16.o g) for 3 h. Then a fresh charge of 10% Pd/C (8.0 g) was added and hydrogenation was continued for another 3 h. The reaction mixture was filtered through celite which was washed with TFE (110 ml) and the filtrate and washings were combined for use in the next synthesis step. A portion was purified by chromatography on silica gel 60 using a LOBAR-prepacked column (Size C). In a typical experiment 4 g of crude material in TFE was loaded onto the column and a sequential step gradient was employed starting with CHCI_{3/}TFE/AcOH (96.5/2.5/1) and ending with MeOH/TFE/AcOH (96.5/2.5/1). The product was pooled and evaporated to 1.9 g of analytically pure product; 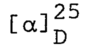 -7.01° (c 0.3, TFE); R 0.36 (CHCl₃/MeOH/ TFE/AcOH; 85/10/2.5/1); mp. 336-338° (dec.).

### (i) Ac-Ser(tBu)-Asp(OtBu)-Ala-Ala-Val-Asp(OtBu)-Thr(tBu)-Ser(tBu)-Ser(tBu)-Glu(OtBul-OC₆H₅

The DMF-containing solution of H-Ala-Val-Asp(OtBu)-Thr(tBu)-Ser(tBu)-Ser(tBu)-Glu(OtBu)-OC₆H₅ (VI, ~16.8 mmol) of Example 6 was cooled to 0° and Ac-Ser(tBu)-Asp(OtBu)-Ala-OSu (VII, 10.92 g, 20.16 mmol, 1.2 eq.) was added. Stirring proceeded at 0° for 30 min. and at 25° for 17 h while the pH was maintained at~8 by addition of Et₃N. Water (2.4 1) was added with stirring and the product was collected by filtration and dried in vacuo. The solid was crushed and washed with CH₃0H (3 x 200 ml) and dried in vacuo. Yield: 23.0 g (91.7%); mp. 255-256°; R_{f} 0.48 (CHCl₃/CH₃OH/AcOH; 80/5/1); 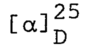 -25.60° (c 1, _{TFE}).

### (j) Ac-Ser(tBu)-Asp(OtBu)-Ala-Ala-Val-Asp(OtBu)-Thr(tBu)-Ser(tBu)-Ser(tBu)-Glu(OtBu)-OH

A solution of Ac-Ser(tBu)-Asp(OtBu)-Ala-Ala-Val-Asp-(OtBu)-Thr(tBu)-Ser(tBu)-Ser(tBu)-Glu(OtBu)-OC₆H₅ (22.73 g, 15.2 mmol) in TFE/H₂O (270 ml/20 ml) was treated with 3% H₂0₂ (15.5 ml, 15.2 mmol, 1 eq.) under magnetic stirring. The reaction mixture was adjusted to pH 10.5 by addition of 1N NaOH and maintained at pH 10.5 over a period of 3 h by further addition of 1N NaOH (triggered by an autotitrator). Then the reaction mixture was adjusted to pH 3 (1N HCl), filtered and the solid was washed with H₂0 (200 ml) and dried in vacuo. Yield: 21.85 g (98.8%); mp. 245° (dec.); 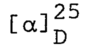 -16.31° (c 0.4, TFE); R_{f} 0.67 (CHC1_{3/}MeOH/ AcOH; 85/10/5).

### (k) Ac-Ser(tBu)-Asp(OtBu)-Ala-Ala-Val-Asp(OtBu)-Thr(tBu)-Ser(tBu)-Ser(tBu)-Glu(OtBu)-Ile-Thr(tBu)-Thr(tBu)-Lys(Boc)-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-OtBu

A solution of H-Ile-Thr(tBu)-Thr(tBu)-Lys(Boc)-Asp-(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-OtBu (8.822 g, 2.932 mmol) and Ac-Ser(tBu)-Asp(OtBu)-Ala-Ala-Val-Asp(OtBu)-Thr(tBu)-Ser(tBu)-Ser(tBu)-Glu(OtBu)-OH (10.82 g, 7.66 mmol, 2.6 eq.) in TFE (230 ml) was treated with HOBt (7.86 g, 51.3 mmol, 10 eq.) and cooled to 0°. DCC (4.49 g, 16.1 mmol, 5.5 eq.) was added to the cold solution under stirring followed by the addition of N-methylmorpholine (5.25 ml, 46.9 mmol, 16 eq.). Stirring proceeded for 1 h at 0° and 23 h at 25°. The reaction mixture was poured into H₂0 (5 1) and the product was filtered, washed with H₂0 (3 x 500 ml), MeOH (3 x 250 ml), DMSO (3 x 250 ml) and MeOH (3 x 250 ml) and dried in vacuo. Yield: 14.985 g TLC shows the presence of some starting materials and a new major spot corresponding to product: R_{f} 0.56 (CHCl₃/MeOH/ AcOH; 85/10/5); R_{f} 0.56 (CHCl₃/MeOH/TFF/AcOH; 85/10/2.5/1). This material can be used directly in the following deprotection step.

### (1) Ac-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-OH, Thymosin α₁

The crude fully protected octacosapeptide (14.985 g) was deprotected with TFA/CH₂C1₂ (65 ml/65 ml) in 6 separate lots under N₂ for 2.5 h at 25°. The reaction mixtures were filtered, the filtrate was poured into ether (1.6 1 total volume) and the resultant precipitate was washed with ether (2 x 200 ml) and dried in vacuo to give 10.274 g of crude thymosin a₁.

A 2.578 g portion of the crude thymosin α₁ was taken up in H20 (10 ml). The pH was adjusted to 8.0 (conc. NH₄0H), the solution was filtered through a 0.45µ millipore filter and applied onto a Whatman Partisil-10 Magnum 20 ODS-3 (2 x 50 cm) column containing C₁₈ reverse phase silica gel (10 µm) which was previously equilibrated with pyridine (3%)/acetic acid (3%). The elution was effected with (flow rate 8 ml/min) pyridine (3%)/acetic acid (3%) for 60 min., followed by gradient elution with 0 to 14% acetonitrile for 60 minutes and finally with pyridine (3%)/acetic acid (3%)/ acetonitrile (14%) for 6 hours. The column was finally stripped with pyridine (3%)/acetic acid (3%)/acetonitrile (acetonitril gradient from 14 to 90%). Fractions were collected at intervals of 2 minutes. The remaining 7.696 g of crude thymosin a₁ were purified in 5 lots by preparative HPLC as described above. A total of 1.170 g (12.84%) of pure thymosin α₁ was prepared by this method. Theoretical yield of thymosin a₁ (based on 2.932 mmol of H-18-peptide) = 9.114 g.

### Example 9

### Synthesis of Desacetylth^{y}mosin a

### (a) Boc-Ser(tBu)-Asp(OtBu)-Ala-OSu

An aqueous solution of Boc-Ser(tBu)-Asp(OtBu)-Ala-OH. CHA (1.205 g, 2.0 mmol) was extracted with a mixture of EtOAc/0.5N H₂SO₄ (10 ml/10 ml). The organic layer was retained, washed with saturated NaCl, dried over MgSO₄ and evaporated in vacuo. The resultant free acid in EtOAc/dioxane (5 ml/10 ml) was treated with HOSu (230 mg, 2.0 mmol, 1 eq.) and cooled to 0°. To the cold solution DCC (412 mg, 2.0 mm, 1 eq.) was added portionwise under stirring and stirring was continued at 0° for 1 h and at 25° for 48 h. The reaction mixture was filtered and the filtrate was evaporated to dryness and crystallized from isopropanol/petroleum ether. Yield: 631 mg (52.5%); mp. 129-136°; 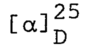 -32.74° (c 1, MeOH); R_{f} 0.86 (CHC1₃/MeOH/ AcOH; 85/10/5); R_{f} 0.79 (CHC1_{3/}MeOH/AcOH; 80/5/2).

### (b) Boc-Ser(tBu)-Asp(OtBu)-Ala-Ala-Val-Asp(OtBu)-Thr(tBu)-Ser(tBu)-Ser(tBu)-Glu(OtBu)-OC6H5

A solution of H-Ala-Val-Asp(OtBu)-Thr(tBu)-Ser(tBu)-Ser(tBu)-Glu(OtBu)-OC6H5 (VI, 0.708 mmol) in DMF (34 ml) was cooled to 0° and Boc-Ser(tBu)-Asp(OtBu)-Ala-OSu (510 mg, 0.85 mmol, 12. eq.) was added. Stirring proceeded at 0° for 30 min. and at 25° for 17 h while the pH was maintained at ~8 by addition of Et₃N. Water (150 ml) was added with stirring and the product was collected by filtration and dried in vacuo. The solid was washed with CH₃OH (2 x 20 ml) and dried in vacuo. Yield: 586 mg (53.3%); mp.> 250°; 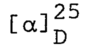 -23.06° (c 1, TFE); R 0.51 (CHCl₃/MeOH/AcOH; 80/2/ 0.4); R_{f} 0.72 (CHC1_{3/}MeOH/AcOH; 80/5/2).

### (c) Boc-Ser(tBu)-Asp(OtBu)-Ala-Val-Val-Asp(OtBu)-Thr(tBu)-Ser(tBu)-Ser(tBu)-Glu(OtBu)-OH

A solution of Boc-Ser(tBu)-Asp(OtBu)-Ala-Val-Val-Asp(OtBu)-Thr(tBu)-Ser(tBu)-Ser(tBu)-Glu(OtBu)-OC₆H₅ (452 mg, 0.29 mmol) in TFE/H₂0 (5.2 ml/0.4 ml) was treated with 3% H₂0₂ (0.31 ml, 0.30 mmol, 1.0 eq.) with magnetic stirring. The reaction mixture was adjusted to pH 10.5 by addition of 1N NaOH (triggered by an autotitrator) and maintained at that pH for 3 h. The reaction mixture was adjusted to pH 1 with 1N HCl, refrigerated, filtered, washed with ^{H}₂⁰ and ^{CH}₃^{0H} and dried in vacuo. Yield: 423 mg (98.4%); mp. 245° (dec.); 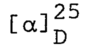 -13.28° (c 1, TFE); R 0.41 (CHCl₃/ MeOH/AcOH); 80/2/0.4).

### (d) H-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-OH (Desacetylthymosin α₁)

A solution of H-Ile-Thr(tBu)-Thr(tBu)-Lys(Boc)-Asp-(otBu)-Leu-Lys(Boc)-Glu(otBu)-Lys(Boc)-Lys(Boc)-Glu(otBu)-Val-Val-Glu(OtBu)-Glu(OtBu)-Ala-Glu(OtBu)-Asn-OtBu (134 mg, 0.0445 mmol) and Boc-Ser(tBu)-Asp(OtBu)-Ala-Val-Val-Asp-(OtBu)-Thr(tBu)-Ser(tBu)-Ser(tBu)-Glu(OtBu)-OH (164 mg, 0.111 mmol, 2.5 eq.) in TFE (4 ml) was treated with HOBt (68 mg, 0.445 mmol, 10 eq.) and cooled to 0°. DCC (50.5 mg, 0.245 mmol, 5.5 eq.) was added to the cold solution under stirring followed by the addition of N-methylmorpholine (to maintain pH 8). Stirring proceeded for 1 h at 0° and for 23 h at 25°. The reaction mixture was poured into H₂0 (50 ml) and the product was filtered, washed with H₂0 (3 x 20 ml), MeOH (3 x 20 ml), DMSO (2 x 20 ml) and MeOH (3 x 20 ml) and dried in vacuo. Yield: 237 mg. The crude fully protected octacosapeptide was deprotected with TFA/CH₂Cl₂ (5 ml/5 ml) under N₂ at 25° for 2.5 h. The reaction mixture was poured into ether (200 ml) and the resultant precipitate was washed with ether (2 x 25 ml) and dried in vacuo to give 185 mg of crude desacetylthymosin α₁.

The crude product was taken up in H₂0 and after pH adjustment to 8.0 with conc. NH₄0H loaded onto an ES Industries Co. column (1.5 x 30 cm, previously equilibrated with Pyr (3%)/AcOH (3%)). Elution of the column was effected with Pyr (3%)/AcOH (3%) for 60 min. which was followed by gradient elution with a 0-13% CH₃CN gradient for the next 60 min. and finally with Pyr (3%)/AcOH (3%)/CH₃CN (13%). Fractions (6 ml each) were collected at 2 min. intervals and aliquots (20 µl) were analyzed by analytical HPLC using a gradient system. Fractions 79-83 were pooled and lyophilized to give pure desacetylthymosin α₁ which was shown to exhibit one major peak by HPLC. The sample was purer than a reference standard.

## Claims

1. A compound of the formula 
R^2-Ser(tBu)-Asp(OtBu)-Ala-Ala-Val-
Asp(OtBu)-Thr(tBu)-Ser(tBu)-Ser(tBu)-
Glu(OtBu)-OR^1
wherein _{R}¹ is hydrogen or phenyl,
_{R}² is acetyl or tert. butyloxycarbonyl and tBu is tert. butyl.

2. A compound as claimed in claim 1 wherein R is hydrogen and _{R}² is acetyl.

3. A compound as claimed in claim 1 wherein R is phenyl and R² is acetyl.

4. A compound as claimed in claim 1 wherein R is hydrogen and R² is tert. butyloxycarbonyl.

5. A compound as claimed in claim 1 wherein R¹ is phenyl and R² is tert. butyloxycarbonyl.

6. A compound of the formula 
R3-Ile-Thr(tBu)-Thr(tBu)-Lys(Boc)-
Asp(OtBu)-Leu-Lys(Boc)-Glu(OtBu)-
Lys(Boc)-Lys(Boc)-Glu(OtBu)-Val-Val-
Glu(OtBu)-Glu(OtBu)-Ala-Glu(OtBu)-Asn-OtBu
wherein _{R}³ is hydrogen or benzyloxycarbonyl, Boc is tert. butyloxycarbonyl and tBu is tert. butyl.

7. A compound as claimed in claim 6 wherein R³ is hydrogen.

8. A compound as claimed in claim 6 wherein _{R}³ is benzyloxycarbonyl.

9. A process for the preparation of thymosin a1 which process comprises coupling the compound of claim 2 with the compound of claim 7 and treating the resulting side chain protected thymosin a₁ with trifluoroacetic acid to remove the protecting groups.

10. A process for the preparation of desacetyl thymosin a₁ which process comprises coupling the compound of claim'4 with the compound of claim 7 and treating the resulting side chain protected desacetyl thymosin a₁ with trifluoroacetic acid to remove the protecting groups.

## Claims (Claims for the following Contracting State(s) : Austria)

1. A process for the preparation of thymosin a₁ which process comprises coupling the compound of formula 
Ac-Ser(tBu)-Asp(OtBu)-Ala-Ala-Val-
Asp(OtBu)-Thr(tBu)-Ser(tBu)-Ser(tBu)-
Glu(OtBu)-OH
 wherein Ac is acetyl and tBu is tert. butyl
with the compound of formula 
H-Ile-Thr(tBu)-Thr(tBu)-Lys(Boc)-Asp(OtBu)-
Leu-Lys(Boc)-Glu(OtBu)-Lys(Boc)-Lys(Boc)-
Glu(OtBu)-Val-Val-Glu(OtBu)-Glu(OtBu)-Ala-
Glu(OtBu)-Asn-OtBu
 wherein tBu is tert. butyl and
Boc is tert. butyloxycarbonyl,
and treating the resulting side chain protected thymosin α₁ with trifluoroacetic acid to remove the protecting groups.

2. A process for the preparation of desacetyl thymosin a₁ which process comprises coupling the compound of formula
Boc-Ser(tBu)-Asp(OtBu)-Ala-Val-Val-Asp(OtBu)-Thr(tBu)-Ser(tBu)-Ser(tBu)-GluLOtBu)-OH
wherein Boc is tert. butyloxycarbonyl and tBu is tert. butyl
with the compound of formula 
H-Ile-Thr(tBu)-Thr(tBu)-Lys(Boc)-Asp(OtBu)-
Leu-Lys(Boc)-Glu(OtBu)-Lys(Boc)-Lys(Boc)-
Glu(OtBu)-Val-Val-Glu(OtBu)-Glu(OtBu)-Ala-
Glu(OtBu)-Asn-OtBu
wherein tBu is tert. butyl and
Boc is tert. butyloxycarbonyl,
and treating the resulting side chain protected desacetyl thymosin α₁ with trifluoroacetic acid to remove the protecting groups.
